# EUROPEAN PATENT APPLICATION

(11) **EP 1 584 923 A2**
(43) Date of publication of application: **12.10.2005**
(21) Application number: 05007345.1
(22) Date of filing: 05.04.2005
(51) Int. Cl.: G01N 33/48, G01N 33/50, C12Q 1/68

(54) **Stabilization of biomolecules in samples**

(30) Priority: 07.04.2004 US 560790 P
(71) Applicant: Roche Diagnostics GmbH, 68305 Mannheim (DE); F.HOFFMANN-LA ROCHE AG, 4070 Basel (CH)
(72) Inventor: Krevolin, Mark D., Pinole, California 94564 (US)

(57) **Abstract**

This invention generally relates to stabilized non-blood-based samples in which microbial biomolecules in non-blood-based samples are stable at high temperatures. The stabilized non-blood-based samples include effective amounts of stabilization components to stabilize the microbial biomolecules in the samples. Stabilization components are typically effective at low levels in these stabilized non-blood-based samples. This provides the advantage of minimizing reagent expenses related to the stabilized non-blood-based samples of the invention. The invention also provides methods of stabilizing microbial biomolecules in non-blood-based samples and related kits.

## Description

### FIELD OF THE INVENTION

The present invention relates generally the stabilization ofbiomolecules in samples and to the analysis ofbiomolecules.

### BACKGROUND OF THE INVENTION

A broad spectrum of microbial organisms, including various bacteria, viruses, fungi, and protozoans are the causative agents of many different infectious diseases. As one bacterial example, *Neisseria gonorrhoeae* are Gram-negative aerobic bacteria that cause gonorrhea in humans. *N. gonorrhoeae* infections, which have a high prevalence and low mortality, are generally acquired by sexual contact and typically affect mucous membranes of the urethra in males and the endocervix in females. However, the infection may also spread to other tissues. The pathogenic mechanism of N. *gonorrhoeae* involves the attachment of the bacterium to nonciliated epithelial cells via pili. The mechanism also includes the production of endotoxin and IgA proteases. In addition, co-infection of N. *gonorrhoeae* and *Chlamydia trachomatis* is frequently observed. Both infections are two known causes of ectopic pregnancy and can also lead to infertility if untreated. They are also known causes of the acute clinical syndromes of mucopurulent cervicitis and pelvic inflammatory disease. Therefore, the detection of N. *gonorrhoeae* and C. *trachomatis* infections, which can be asymptomatic, especially in females, is of consequence to individuals in need of treatment and to broader populations at risk of acquiring and further propagating the infections.

The detection and identification of, for example, bacterial infections has traditionally been accomplished by pure culture isolation and determination procedures that make use of knowledge of specimen source, growth requirements, visible growth features, microscopic morphology, staining reactions, and biochemical characteristics. To illustrate, some of these methods of detecting and identifying N. *gonorrhoeae* infections, include Gram-staining, culturing on selective agar media, and cytochrome oxidase and carbohydrate utilization testing. Serological assays, including co-agglutination and fluorescent antibody staining have also been described for the detection of N. *gonorrhoeae.* Culture-based methods, while relatively sensitive, are generally slow to perform, often including overnight incubation, and are labor intensive. The Gram-stain and antibody-based tests typically provide results in less than one hour, but are generally of lower sensitivity than culture-based methods.

The use of specific polynucleotide sequences as probes for the recognition of infectious agents is one alternative to problematic immunological identification assays and other methodologies. For example, nucleic acid probes complementary to targeted nucleic acid sequences have been used in hybridization procedures, such as Southern blots and dot blots, to detect the target nucleic acid sequence. Many of these hybridization procedures have depended on the cultivation and/or enrichment of the organism and, thus, are unsuitable for rapid diagnosis. The advent of techniques for the rapid amplification of specific nucleic acid sequences, such as the polymerase chain reaction, among many others, has provided a mechanism to use sequence specific probes directly on clinical specimens, thereby eliminating enrichment and *in vitro* culturing of the pathogen prior to performing the hybridization assay. Thus, nucleic acid amplification tests can provide simple and rapid diagnostic techniques for the detection of pathogens in clinical samples.

It is not always possible to immediately test samples or specimens derived from patients for the presence of infectious agents using, e.g., nucleic acid amplification-based hybridization assays. For example, diagnostic laboratories may not be located at the point of care and accordingly, samples must be transported between these sites. This creates a time lag between the point at which samples are taken and the point at which samples are actually analyzed. Moreover, even if sample analysis is performed at the point of care, sample backlogs may cause testing delays. As biomolecules in specimens generally degrade over time, delays such as these can compromise the accuracy of the particular assay. To illustrate, human urine is commonly used for the diagnostic testing of many chemicals, metabolites, enzymes, and organisms. Urine typically includes various components, such as nucleases and proteases, involved in the breakdown of biomolecules such as nucleic acids and proteins. With improper handling of urine or other samples, diagnostic results may be biased due to analyte degradation, including the potentially dangerous situation of obtaining false negatives for patients.

To minimize the occurrence of sample degradation, biomolecules are generally preserved or stabilized for subsequent analysis. For example, samples, such as urine specimens are often either maintained at low temperatures such as 8°C or below and/or are chemically preserved with stabilization components that frequently include chelating agents to reduce the activity of, e.g., proteases and nucleases in an effort to maintain the integrity of target biomolecules of the particular diagnostic tests to be utilized. In addition, pre-existing approaches to chemical stabilization often include the use of high levels of other reagents such as chaotropic agents. Disadvantages of these pre-existing special handling approaches to biomolecule stabilization include the expense and time consumed in maintaining low temperatures, and the costs incurred using, e.g., chelating agents and high levels of chaotropic agents.

The present invention provides methods of stabilizing microbial biomolecules in samples, which avoid the disadvantages of many of these pre-existing approaches to stabilizing biomolecules. These and other features of the invention will be apparent upon complete review of the following disclosure.

### SUMMARY OF THE INVENTION

The present invention relates generally to the stabilization of biomolecules in samples. The approaches to biomolecule stabilization of the invention generally avoid certain special handling requirements of many pre-existing techniques. For example, biomolecules are typically stabilized at higher temperatures (e.g., at room temperature, etc.) than are utilized with certain pre-existing techniques to minimize the degradation of biomolecules in samples. The high temperature techniques described herein typically reduce the overall expense associated with, and otherwise facilitate, stabilizing biomolecules in samples relative to these pre-existing approaches. Furthermore, the stabilization techniques of the present invention generally include the use of smaller amounts of chaotropic reagents than are used by many pre-existing methodologies. Accordingly, this typically further reduces the overall expense incurred when stabilizing biomolecules relative to these pre-existing approaches. In addition, the stabilization of biomolecules at high temperatures while using lower amounts of chaotropic reagents is one surprising result of the present invention. Moreover, costs of stabilizing biomolecules in samples are also typically minimized relative to other approaches by not using chelating agents in certain embodiments of the invention. In addition to methods of stabilizing microbial biomolecules in non-blood-based samples, the invention also provides stabilized non-blood-based samples and kits for stabilizing microbial biomolecules in non-blood-based samples.

In one aspect, the present invention provides a stabilized non-blood-based sample that includes at least one non-blood-based sample that comprises microbial biomolecules, and an effective amount of at least one stabilization component comprising at least one chaotropic reagent in which the microbial biomolecules in the stabilized non-blood-based sample are stable at a temperature of about 20°C or more. In certain embodiments, for example, levels (e.g., activity levels or the like) of the microbial biomolecules in the stabilized non-blood-based sample deviate from one another by less than about 15% over at least about a seven day period whether a temperature of the stabilized non-blood-based sample is maintained at about 30°C or between about 8°C and about 2°C. Optionally, the stabilization component includes one or more substances in addition to the chaotropic reagent including, e.g., organic solvents, reducing agents, buffers, detergents, and/or the like. The stabilization component is typically substantially free of a chelating agent (e.g., lacks a chelating agent, etc.). In certain embodiments, the stabilized non-blood-based sample further includes at least one transport medium. Although many different types of non-blood-based samples are optionally utilized in the stabilized non-blood-based sample, the non-blood-based sample comprises urine in some embodiments of the invention. In these embodiments, the chaotropic reagent is typically present in the stabilized non-blood-based sample at a concentration of about 0.5M or less.

In another aspect, the invention provides a reaction mixture that includes a stabilized non-blood based sample and at least one nucleic acid amplification reagent. The stabilized non-blood based sample includes at least one non-blood-based sample that includes one or more microbial nucleic acids, and an effective amount of at least one stabilization component that includes at least one chaotropic reagent. The stabilization component stabilizes the microbial nucleic acids at a temperature of about 20°C or more. The nucleic acid amplification reagent includes one or more of, e.g., a primer nucleic acid, a probe nucleic acid, a nucleotide incorporating biocatalyst, extendible nucleotides, or the like. Exemplary microbial nucleic acids include *Neisseria gonorrhoeae* nucleic acids, *Neisseria meningitidis* nucleic acids, papillomaviral nucleic acids, *Plasmodium falciparum* nucleic acids, *Chlamydia muridarum* nucleic acids, *Chlamydia trachomatis* nucleic acids, and/or the like. The effective amount of the stabilization component generally includes about 45% or less of a total weight of the stabilized non-blood-based sample. In certain embodiments, the stabilization component further includes one or more of, e.g., an organic solvent, a reducing agent, a buffer, a detergent, or the like. Typically, the stabilization component is substantially free of a chelating agent.

In another aspect, the invention relates to a method of stabilizing microbial biomolecules in non-blood-based samples. The method includes providing at least one non-blood-based sample comprising the microbial biomolecules (e.g., nucleic acids, DNAs, RNAs, polypeptides, proteins, peptides, prions, and/or the like). The method also includes contacting the non-blood-based sample with an effective amount of at least one stabilization component comprising at least one chaotropic reagent to produce a stabilized non-blood-based sample in which the stabilization component stabilizes the microbial biomolecules at a temperature of about 20°C or more. The effective amount of the stabilization component generally comprises about 45% or less of a total weight of the stabilized non-blood-based sample. In some embodiments, for example, the non-blood-based sample comprises urine and the effective amount of the stabilization component comprises about 15% or less of a total weight of the stabilized non-blood-based sample. The stabilization component is generally substantially free of a chelating agent (e.g., lacks a chelating agent, etc.). In some embodiments, levels of the microbial biomolecules in the stabilized non-blood-based sample deviate from one another by less than about 15% over at least about a seven day period whether a temperature of the stabilized non-blood-based sample is maintained at about 30°C or between about 8°C and about 2°C. In some embodiments, the method further includes contacting the non-blood-based sample with at least one transport medium.

In certain embodiments, the method further includes isolating one or more of the microbial biomolecules after the contacting step. Typically, the method further includes detecting one or more of the microbial biomolecules even if one or more of the microbial biomolecules are not isolated after the contacting step. To illustrate, the detecting step optionally comprises amplifying a detectable signal produced by the microbial biomolecules, or by a microbial biomolecule detection reagent that detectably binds to at least one of the microbial biomolecules, to produce an amplified signal, and detecting the amplified signal. To further illustrate, the microbial biomolecules optionally comprise nucleic acids and the detecting step optionally comprises amplifying at least a segment of one or more of the nucleic acids to produce amplified nucleic acids, and detecting one or more of the amplified nucleic acids during or after the amplifying step.

In still another aspect, the invention provides a kit that includes at least one stabilization component comprising at least one chaotropic reagent. The kit also includes instructions for contacting at least one non-blood-based sample comprising microbial biomolecules with an effective amount of the stabilization component to produce a stabilized non-blood-based sample in which the microbial biomolecules are stable at a temperature of about 20°C or more. The stabilization component is generally substantially free of a chelating agent (e.g., lacks a chelating agent, etc.). In some embodiments, the stabilization component comprises a stabilization solution. Optionally, the stabilization component comprises one or more solid materials. In certain embodiments, the kit further includes at least one container that comprises the stabilization component. In some embodiments, the kit further includes at least one transport medium. Optionally, the kit further includes instructions for detecting the microbial biomolecules in the stabilized non-blood-based sample.

### BRIEF DESCRIPTION OF THE DRAWINGS

Figure 1 is a graph showing N. *gonorrhoeae* activity levels detected in urine samples under various conditions at different timepoints.
Figure 2 is a graph of the data presented in Figure 1 that shows actual total optical density readings for the samples.

### DETAILED DESCRIPTION OF THE INVENTION

### I. DEFINITIONS

Before describing the present invention in detail, it is to be understood that this invention is not limited to particular embodiments. It is also to be understood that the terminology used herein is for the purpose of describing particular embodiments only, and is not intended to be limiting. Units, prefixes, and symbols are denoted in the forms suggested by the International System of Units (SI), unless specified otherwise. Numeric ranges are inclusive of the numbers defining the range. As used in this specification and the appended claims, the singular forms "a", "an" and "the" also include plural referents unless the context clearly dictates otherwise. Thus, for example, reference to "a biomolecule" also includes two or more biomolecules, and the like. Further, unless defined otherwise, all technical and scientific terms used herein have the same meaning as commonly understood by one of ordinary skill in the art to which the invention pertains. The terms defined below, and grammatical variants thereof, are more fully defined by reference to the specification in its entirety.

A "5'-nuclease probe" refers to an oligonucleotide probe that comprises at least two labels and emits radiation of increased intensity after one of the two labels is cleaved or otherwise separated from the probe. In certain embodiments, for example, a 5'-nuclease probe is labeled with two different fluorescent dyes, e.g., a 5' terminus reporter dye and the 3' terminus quenching dye or moiety. When the probe is intact, energy transfer typically occurs between the two fluorophores such that fluorescent emission from the reporter dye is quenched. During an extension step of a polymerase chain reaction, for example, a 5'-nuclease probe bound to a template nucleic acid is cleaved by the 5' nuclease activity of, e.g., a Taq polymerase such that the fluorescent emission of the reporter dye is no longer quenched. Exemplary 5'-nuclease probes are described in, e.g., U.S. Pat. No. 5,210,015, entitled "HOMOGENEOUS ASSAY SYSTEM USING THE NUCLEASE ACTIVITY OF A NUCLEIC ACID POLYMERASE," issued May 11, 1993 to Gelfand et al., U.S. Pat. No. 5,994,056, entitled "HOMOGENEOUS METHODS FOR NUCLEIC ACID AMPLIFICATION AND DETECTION," issued November 30, 1999 to Higuchi, and U.S. Pat. No. 6,171,785, entitled "METHODS AND DEVICES FOR HEMOGENEOUS NUCLEIC ACID AMPLIFICATION AND DETECTOR," issued January 9, 2001 to Higuchi, which are each incorporated by reference.

The term "biomolecule" refers to an organic molecule comprised by, or derived or originating from, an organism or particle (e.g., a viral particle). These include, for example, nucleotides, amino acids, sugars, fatty acids, steroids, nucleic acids, polypeptides, peptides, peptide fragments, carbohydrates, lipids, and combinations of these (e.g., glycoproteins, ribonucleoproteins, lipoproteins, or the like).

The term "blood" refers to a substance obtained directly from the cardiovascular system of an organism, e.g., via venipuncture.

The term "buffer" refers to a substance or mixture of substances that is resistant to changes in pH. Illustrative buffers include tris-(hydroxymethyl)aminoethane (TRIS), tris-(hydroxymethyl)aminomethane sodium phosphate, sodium acetate, sodium citrate, and the like.

The term "chaotropic reagent" refers to a reagent that can disrupt hydrophobic interactions. For example, chaotropic reagents can denature or solubilize proteins. Exemplary chaotropic reagents include guanidine isothiocyanate or a salt thereof, guanidine hydrochloride or a salt thereof, and the like.

The term "chelating agent" refers to a substance that binds metal ions. Exemplary chelating agents include ethylenediaminetetraacetic acid (EDTA), [ethylenebis(oxyethylenenitrilo)]tetraacetic acid (EGTA), 1,2-bis(2-aminophenoxy)ethane-N,N,N', N'-tetraacetic acid (BAPTA), and the like.

The term "detectably binds" refers to binding (e.g., covalent or non-covalent binding) between at least two molecular species (e.g., a probe nucleic acid and a target nucleic acid, an antibody and a target biomolecule, etc.) that is detectable above a background signal (e.g., noise) using one or more methods of detection.

The term "detergent" refers to a substance that comprises both hydrophilic and hydrophobic moieties (i.e., is an amphiphile). Detergents are typically non-ionic, ionic, or zwitterionic. Exemplary detergents include polyoxyethylenesorbitan monolaurate (TWEEN® 20), sodium dodecyl sulfate, and the like.

The term "effective amount" of a component refers to an amount of the component that is sufficient to cause a desired effect, such as the stabilization of biomolecules in a sample. In certain embodiments of the invention, for example, a stabilization component that is about 45% or less of a total weight of a given stabilized non-blood-based sample is an amount that is sufficient to stabilize biomolecules in the mixture.

An "extendible nucleotide" refers to a nucleotide to which at least one other nucleotide can be added or covalently bonded, e.g., in a reaction catalyzed by a nucleotide incorporating biocatalyst once the extendible nucleotide is incorporated into a nucleotide polymer. Examples of extendible nucleotides include deoxyribonucleotides and ribonucleotides. An extendible nucleotide is typically extended by adding another nucleotide at a 3'-position of the sugar moiety of the extendible nucleotide.

The term "high temperature" refers to a temperature that is about 20°C or more. To illustrate, stabilized non-blood-based samples of the invention are typically maintained at temperatures between about 20°C and about 40°C, more typically maintained at temperatures between about 21°C and about 35°C, and still more typically maintained at temperatures between about 22°C and about 30°C (e.g., at about room temperature).

A "lysis reagent" refers to a reagent that effects or facilitates the lysis of cells in a mixture.

The term "microbe" refers to any microscopic or submicroscopic organism or particle. Microbes include, for example, bacteria, virsuses, protozoans, and certain fungi.

The term "microbial biomolecule" refers to a biomolecule that originates from a microbe. Microbial biomolecules include, for example, bacterial biomolecules, viral biomolecules, fungal biomolecules, protozoal biomolecules, or the like.

The term "microbial biomolecule detection reagent" refers to a reagent that detectably binds (e.g., hydrogen bonds in nucleic acid hybridization, in antibody-antigen recognition, or other types of binding interactions) to a target biomolecule. Exemplary microbial biomolecule detection reagents include, e.g., oligonucleotide primers and probes, antibodies, and the like.

The term "microbial nucleic acid" refers to a nucleic acid that originates from a microbe. Microbial nucleic acids include, for example, bacterial nucleic acids, viral nucleic acids, fungal nucleic acids, protozoal nucleic acids, or the like.

A "mixture" refers to a combination of two or more different components.

A "non-blood-based sample" refers to a sample obtained from a subject (e.g., a mammalian subject, particularly a human subject) that includes one or more substances other than blood. Exemplary non-blood-based samples include urine, seminal fluid, seminal plasma, prostatic fluid, vaginal fluid, vaginal scrapings, cervical fluid, uterine fluid, cervical scrapings, amniotic fluid, anal scrapings, feces, digestive fluid, nipple secretions, lymph, lung/bronchial washes, mucus, sputum, tears, buckle cells, saliva, tissue samples, spinal fluid, perspiration, and the like. In certain embodiments, non-blood-based samples obtained from different subjects are pooled together and stabilized as described herein before being subjected to one or more screening protocols.

The term "nucleic acid" encompasses any physical string of monomer units that can be corresponded to a string of nucleotides, including a polymer of nucleotides (e.g., a typical DNA or RNA polymer), PNAs, modified oligonucleotides (e.g., oligonucleotides comprising nucleotides that are not typical to biological RNA or DNA in solution, such as 2'-O-methylated oligonucleotides), and/or the like. A nucleic acid can be, e.g., single-stranded or double-stranded. Unless otherwise indicated, a particular nucleic acid sequence of this invention encompasses complementary sequences, in addition to the sequence explicitly indicated. Nucleic acids can also include modifications such as labels, quenchers, or the like. The term nucleic acid is used interchangeably with, e.g., oligonucleotide, polynucleotide, gene, cDNA, and mRNA encoded by a gene.

A "nucleic acid amplification reagent" refers to a reagent that can participate in and/or facilitate a nucleic acid amplification reaction and/or the detection of nucleic acid amplification reaction products. Exemplary nucleic acid amplification reagents include primer nucleic acids, probe nucleic acids, nucleotide incorporating biocatalysts, extendible nucleotides, etc.

A "nucleotide incorporating biocatalyst" refers to a catalyst that catalyzes the incorporation of nucleotides into a nucleic acid. Nucleotide incorporating biocatalysts are typically enzymes. An "enzyme" is a protein- and/or nucleic acid-based catalyst that acts to reduce the activation energy of a chemical reaction involving other compounds or "substrates." A "nucleotide incorporating enzyme" refers to an enzyme that catalyzes the incorporation of nucleotides into a nucleic acid. Exemplary nucleotide incorporating enzymes include, e.g., DNA polymerases, RNA polymerases, terminal transferases, reverse transcriptases, telomerases, polynucleotide phosphorylases, and the like. A "thermostable enzyme" refers to an enzyme that is stable to heat (i.e., resists breakdown or denaturation) and retains sufficient catalytic activity when subjected to elevated temperatures for selected periods of time. For example, a thermostable polymerase retains sufficient activity to effect subsequent primer extension reactions when subjected to elevated temperatures for the time necessary to effect denaturation of double-stranded nucleic acids. Heating conditions necessary for nucleic acid denaturation are well known in the art and are exemplified in U.S. Pat. Nos. 4,683,202 and 4,683,195, which are both incorporated by reference. As used herein, a thermostable polymerase is typically suitable for use in a temperature cycling reaction such as the polymerase chain reaction ("PCR"), etc. For a thermostable nucleotide incorporating enzyme, enzymatic activity refers to the catalysis of the combination of the nucleotides in the proper manner to form primer nucleic acid extension products that are complementary to a template nucleic acid. Other thermostable enzymes referred to herein, include thermostable pyrophosphatases, which similarly retain sufficient activity when subjected to elevated temperatures, e.g., to minimize pyrophosphorolysis.

The term "polypeptide" refers to a polymer comprising two or more amino acid residues (e.g., a peptide or a protein). The polymer can additionally comprise non-amino acid elements such as labels, quenchers, blocking groups, or the like and can optionally comprise modifications such as glycosylation or the like. The amino acid residues of a polypeptide can be natural or non-natural and can be unsubstituted, unmodified, substituted, or modified.

A "primer nucleic acid" or "primer" is typically a nucleic acid that can hybridize to a template or target nucleic acid and permit chain extension or elongation using, e.g., a nucleotide incorporating biocatalyst, such as a thermostable polymerase under appropriate reaction conditions. A primer nucleic acid is typically a natural or synthetic oligonucleotide (e.g., a single-stranded oligodeoxyribonucleotide, etc.). Although other primer nucleic acid lengths are optionally utilized, they typically range from 15 to 35 nucleotides. Short primer nucleic acids generally utilize cooler temperatures to form sufficiently stable hybrid complexes with template nucleic acids. A primer nucleic acid that is at least partially complementary to a subsequence of a template nucleic acid is typically sufficient to hybridize with the template nucleic acid for extension to occur. A primer nucleic acid can be labeled, if desired, by incorporating a label detectable by, e.g., spectroscopic, photochemical, biochemical, immunochemical, or chemical techniques. To illustrate, useful labels include radioisotopes, fluorescent dyes, electron-dense reagents, enzymes (as commonly used in ELISAs), biotin, or haptens and proteins for which antisera or monoclonal antibodies are available. Many of these and other labels are described further herein and/or are otherwise known in the art.

The term "probe nucleic acid" or "probe" refers to a labeled or unlabeled oligonucleotide capable of selectively hybridizing to a target nucleic acid under suitable conditions. Typically, a probe is sufficiently complementary to a specific target sequence (e.g., a bacterial nucleic acid, a viral nucleic acid, a fungal nucleic acid, a protozoal nucleic acid, or the like) contained in a nucleic acid sample to form a stable hybridization duplex with the target sequence under a selected hybridization condition, such as, but not limited to, a stringent hybridization condition. A hybridization assay carried out using the probe under sufficiently stringent hybridization conditions permits the selective detection of a specific target sequence. The term "hybridizing region" refers to that region of a nucleic acid that is exactly or substantially complementary to, and therefore hybridizes to, the target sequence. For use in a hybridization assay for the discrimination of single nucleotide differences in sequence, the hybridizing region is typically from about 8 to about 100 nucleotides in length. Although the hybridizing region generally refers to the entire oligonucleotide, the probe may include additional nucleotide sequences that function, for example, as linker binding sites to provide a site for attaching the probe sequence to a solid support or the like. In certain embodiments, an probe nucleic acid comprises one or more labels (e.g., a reporter dye, a quencher moiety, etc.), such as a 5'-nuclease probe, a FRET probe, a molecular beacon, or the like, which can also be utilized to detect hybridization between the probe and target nucleic acids in a sample. In some embodiments, the hybridizing region of the probe nucleic acid is completely complementary to the target sequence. However, in general, complete complementarity is not necessary; stable duplexes may contain mismatched bases or unmatched bases. Modification of the stringent conditions may be necessary to permit a stable hybridization duplex with one or more base pair mismatches or unmatched bases. Sambrook et al., Molecular Cloning: A Laboratory Manual, 3rd Ed., Cold Spring Harbor Laboratory Press, Cold Spring Harbor, N.Y. (2001), which is incorporated by reference, provides guidance for suitable modification. Stability of the target/probe duplex depends on a number of variables including length of the oligonucleotide, base composition and sequence of the oligonucleotide, temperature, and ionic conditions. One of skill in the art will recognize that, in general, the exact complement of a given probe is similarly useful as a probe. One of skill in the art will also recognize that, in certain embodiments, probe nucleic acids can also be used as primer nucleic acids.

The term "organic solvent" refers to an organic chemical substance or mixture of organic chemical substances that dissolve or dissolve in other organic matter to form a solution. Illustrative organic solvents include alcohols, polyethylene glycol, acetone, and the like.

A "reaction mixture" refers a mixture that comprises molecules that can participate in and/or facilitate a given reaction. For example, a nucleic acid amplification reaction mixture includes components necessary for a nucleic acid amplification reaction. Thus, a nucleic acid amplification reaction mixture is suitable for use in a nucleic acid amplification method for amplifying a target nucleic acid, although the reaction mixture may initially be incomplete, so that the initiation of the nucleic acid amplification reaction is controlled by the user. In this manner, the reaction may be initiated once a final component, such as a nucleotide incorporate biocatalyst (e.g., a polymerase, a ligase, and/or the like), is added, to provide a complete nucleic acid amplification reaction mixture. Typically, a nucleic acid amplification reaction mixture will contain a buffer, suitable for polymerization activity, extendible nucleotides, and primer nucleic acids. In certain embodiments, probe nucleic acids (e.g., 5'-nuclease probes, etc.) are also included to monitor the progression of a nucleic acid amplification reaction.

The term "reducing agent" refers to a substance that reduces a chemical composition typically by donating electrons. In certain embodiments, reducing agent effect cleavage of disulfide bonds in proteins. Exemplary reducing agents include dithiothreitol (DTT), dithioerithritol, and the like.

A biomolecule is "stable" or "preserved" in a stabilized non-blood-based sample when levels (e.g., activity levels (e.g. measure by optical density [OD] or the like), etc.) of the biomolecule (e.g., microbial biomolecules or the like) in the mixture deviate from one another by less than about 20% (e.g., about 15%, about 10%, about 5%, etc.) over at least about a seven day period (e.g., seven consecutive days, etc.) whether a temperature of the mixture is maintained at about 30°C or between about 8°C and about 2°C.

The term "stabilization component" refers to a substance or mixture of substances that stabilize or preserve biomolecules in a mixture, e.g., preventing those biomolecules from being degraded in the mixture or at least minimizing such degradation. In certain embodiments of the invention, stabilization components comprise lysis reagents.

A "stabilized non-blood-based sample" refers to a mixture that comprises at least one non-blood-based sample that is suspected to include one or more microbial biomolecules and an effective amount of at least one stabilization component that includes at least one chaotropic agent in which the microbial biomolecules are stable at a temperature of 20°C ore more. In certain embodiments, for example, microbial nucleic acids are preserved in stabilized non-blood-based samples prior to being detected, e.g., via a nucleic acid amplification reaction.

The term "transport medium" refers to a medium that is used for the collection, transport, and/or storage of samples. Transport media are typically provided in a liquid-or solid-phase, and are generally either specific or non-specific for a particular organism.

Exemplary transport media include, e.g., M4, JEMBEC® media, Transgrow media, and the like.

### II. INTRODUCTION

The present invention relates generally to the stabilization of biomolecules in samples. In certain embodiments, for example, microbial biomolecules are preserved or stabilized in samples derived from patients until those biomolecules can be analyzed to provide diagnoses for those patients. Certain pre-existing techniques attempt to minimize biomolecule degradation by storing samples comprising those biomolecules at lower temperatures, e.g., between about 8°C and about 2°C. In contrast, biomolecules preserved as described herein are stable even at higher temperatures, such as at ambient or room temperatures. Thus, the approaches to biomolecule stabilization of the invention are not limited by the special handling requirements of these pre-existing techniques. Moreover, the approaches to stabilizing biomolecules provided by the present invention typically involve the use of smaller amounts, if any, of various stabilization components (e.g., chaotropic reagents, chelating agents, etc.) than are commonly utilized with other biomolecule preservation techniques. Accordingly, the costs associated with stabilizing biomolecules, as described herein, are typically less than those associated with many of these pre-existing approaches to biomolecule stabilization to illustrate some of the advantages of the present invention. Examples that illustrate certain of these advantages are provided below.

In certain embodiments, the invention provides stabilized non-blood-based samples and kits for stabilizing microbial biomolecules in non-blood-based samples. In addition, the invention also provides methods of stabilizing microbial biomolecules in such samples.

### III. STABILIZED NON-BLOOD-BASED SAMPLES AND REACTION MIXTURES

The invention provides stabilized non-blood-based samples in which microbial biomolecules are stable at high temperatures (e.g., at about 20°C or more). For example, activity levels (e.g., measured by OD or the like) of microbial biomolecules in a stabilized non-blood-based sample of the invention generally deviate from one another by less than about 20% over at least about a seven day period whether a temperature of the stabilized non-blood-based sample is maintained at about 30°C or between about 8°C and about 2°C. The stabilized non-blood-based samples include non-blood-based samples that comprise the microbial biomolecules, and effective amounts of stabilization components that include chaotropic reagents. The stabilized non-blood-based samples of the invention are typically utilized in various molecular analyses, such as nucleic acid amplification-based diagnostic testing, among many others assays. The reaction mixtures of the invention include stabilized non-blood-based samples and nucleic acid amplification reagents.

### A. NON-BLOOD-BASED SAMPLES

Although urine is emphasized herein for clarity of illustration, it will be appreciated that essentially any non-blood-based sample obtained from a subject is optionally utilized in the stabilized non-blood-based samples and other aspects of the invention. Other exemplary non-blood-based samples include, e.g., seminal fluid, seminal plasma, prostatic fluid, vaginal fluid, vaginal scrapings, cervical fluid, uterine fluid, cervical scrapings, amniotic fluid, anal scrapings, feces, digestive fluid, nipple secretions, lymph, lung/bronchial washes, mucus, sputum, tears, buckle cells, saliva, tissue samples, spinal fluid, perspiration, and/or the like. In addition, many different types of microbial biomolecules are stabilized in the stabilized non-blood-based samples of the invention including, e.g., nucleic acids, DNAs, RNAs, polypeptides, proteins, peptides, prions, and/or the like. The sources of these microbial biomolecules are widely varied, but typically include those that are infectious agents in mammals, particularly in humans. Examples of these microbial sources include, e.g., bacteria, viruses, fungi, protozoans, and the like. To further illustrate, exemplary bacterial sources include, e.g., *Neisseria gonorrhoeae*, *Neisseria meningitidis Chlamydia muridarum, Chlamydia trachomatis,* and the like. An exemplary viral source is the papilloma virus, whereas examples of fungal sources include *Histoplasma capsulatus, Blastomyces dematitidis,* etc. One exemplary protozoal source is *Plasmodium falciparum,* which causes malaria in humans. Many other microbial sources are well known in the art.

Non-blood-based samples are obtained using essentially any collection technique. In some embodiments, for example, urine samples are collected from patients, whereas in others, swabs, brushes, or loops are utilized to take samples from, e.g., the endocervix, the urethra, the rectum, the vagina, the oropharynx, an eye, etc. of the particular patient. A number of swab types are suitable for collecting these non-blood-based samples, including rayon fibre-tipped swabs, cotton swabs, DACRON® swabs, or the like. Optionally, swabs are coated (e.g., serum or bovine albumin-coated swabs, calcium-alginate swabs, etc.). Urine or swabbed specimens are typically placed into sample containers, such as polypropylene tubes, screwed-cap sarstedt tubes, and the like. The stabilization components of the invention are generally added to these samples containers before or after the particular non-blood-based sample is added to the containers as part of a sample processing protocol. Stabilization components are described in greater detail below. In certain embodiments, urine samples are contacted with a wash buffer, centrifuged, and the supernatant removed before the stabilization component is contacted with the remaining sample. Specimen collection and handling techniques are also described in, e.g., Forrest et al. (Eds.), Manual of Clinical Microbiology, 8^{th} Edition, ASM Press (2003), Murray et al. (Eds.), Manual of Clinical Microbiology, 7^{th} Edition, ASM Press (1999), and Forbes et al., Bailey and Scott's Diagnostic Microbiology, 11^{th} Edition, Elsevier Science (2002), which are each incorporated by reference. Methods of storing specimens, culturing cells, isolating and preparing biomolecules (e.g., nucleic acids, proteins, etc.) from these sources are generally known in the art and many of these are described further in the references and/or examples provided herein.

In certain embodiments, the sample container also includes one or more additional components, such as a specimen diluent, a transport medium suitable for the target microbe, and/or the like. Suitable transport media for N. *gonorrhoeae,* for example, include non-nutrient transport media, such as Stuart or Amies media, or selective growth and transport media, such as M4, JEMBEC® , or Transgrow media. Other exemplary transport media are described herein. Many different types of transport media are commercially available from various suppliers including, e.g., Quelabs Laboratories, Inc. (Montreal, Canada). Transport media and media systems are also described in, e.g., U.S. Pat. No. 5,096,062, entitled "TRANSPORT SYSTEM FOR SHIPPING MICROBIOLOGICAL SAMPLES" issued March 17, 1992 to Burkardt et al., U.S. Pat. No. 5,545,555, entitled "MICROBIAL TRANSPORT MEDIA" issued August 13,1996 to Racioppi et al., and U.S. Pat. No. 5,702,944, entitled "MICROBIAL TRANSPORT MEDIA" issued December 30, 1997 to Racioppi et al., which are each incorporated by reference.

### B. STABILIZATION COMPONENTS

The stabilization components used in the stabilized non-blood-based samples of the invention include chaotropic reagents, which inactivate enzymes, such as nucleases (e.g., DNases, RNases, etc.) and proteases (e.g., serine proteases, aspartic proteases, cysteine proteases, metalloproteases, etc.) by causing them to unfold. In the stabilized non-blood-based samples of the invention, this inactivation effects microbial biomolecule stabilization, as these enzymes are inhibited or prevented from degrading biomolecules in the mixture even at high temperatures. The stabilization components described herein are typically added to samples upon sample collection. In addition to chaotropic reagents, the stabilization components utilized as described herein include additional compounds, such as organic solvents, reducing agents, buffers, and/or detergents in some embodiments. Although exemplary amounts of these additional compounds are indicated herein for purposes of illustration, it will be appreciated by persons of skill in the art that other amounts can also be added to the stabilization components in these embodiments. An exemplary stabilization component is also described in the examples provided below. Chaotropic reagents and other compounds used in these stabilization components are generally readily available from various commercial suppliers including, e.g., Sigma-Aldrich, Inc. (St. Louis, MO, USA). Unlike certain pre-existing approaches to biomolecule stabilization, the stabilization components of the present invention typically lack chelating agents, such as EDTA, EGTA, BAPTA, and the like. This effects a cost savings relative to these other approaches among other advantages. An effective amount of a stabilization component typically comprises about 45% or less of a total weight of the stabilized non-blood-based sample, more typically about 35% or less of a total weight of the stabilized non-blood-based sample, and still more typically about 25% or less of a total weight of the stabilized non-blood-based sample. For example, when the non-blood-based sample is urine, the effective amount of the stabilization component typically comprises about 15% or less (e.g., about 12%, about 10%, about 8%, etc.) of a total weight of the stabilized non-blood-based sample.

### Chaotropic Reagents

Among the surprising results of the stabilized non-blood-based samples and other aspects of the invention is that microbial biomolecules are stabilized using low amounts of chaotropic reagents while the stabilized non-blood-based samples are maintained at high temperatures. In certain embodiments, for example, chaotropic reagents are present in stabilized non-blood-based samples at concentrations of about 5.0M or less (e.g., about 4.5M, about 4.0M, about 3.5M, etc). To further illustrate, when the non-blood-based sample is urine, the chaotropic reagent is typically present in the stabilized non-blood-based sample at a concentration of about 0.5M or less (e.g., about 0.45M, about 0.40M, about 0.35M, etc.). Chaotropic reagents can disrupt hydrophobic interactions, e.g., causing the denaturation or solubilization of proteins. Chaotropic agents in the stabilization components described herein effect biomolecule stabilization by, e.g., denaturing nucleases, proteases, or the like in samples, thereby substantially preventing those enzymes from degrading target biomolecules (e.g., nucleic acids, proteins, etc.) in the stabilized non-blood-based samples of the invention. Exemplary chaotropic reagents that are optionally utilized include one or more of, e.g., guanidine isothiocyanate or a salt thereof, guanidine hydrochloride or a salt thereof, urea, sodium iodide, potassium iodide, potassium chloride, ammonium thiocyanate, sodium thiocyanate, trifluoroethanol, sodium perchlorate, or the like.

### Organic Solvents

In certain embodiments, stabilization components further include at least one organic solvent. In these embodiments, the organic solvent typically comprises between about 0.1% and about 10% (e.g., about 0.5%, about 1%, about 3%, about 5%, about 7%, about 9%, etc.) of a total weight of the stabilized non-blood-based sample. For example, when the non-blood-based sample comprises urine, the organic solvent optionally includes between about 0.1% and about 1% (e.g., about 0.3%, about 0.5%, about 0.7%, about 0.9%, etc.) of a total weight of the stabilized non-blood-based sample. Certain exemplary organic solvents optionally utilized in the stabilization components of the invention include one or more of, e.g., polyethylene glycol, acetone, methanol, ethanol, dimethylsulfoxide, or the like. Typically, the polyethylene glycol comprises a molecular weight of between about 5000 daltons and about 15000 daltons.

### Reducing Agents

In some embodiments, stabilization components further comprise at least one reducing agent. Typically, the reducing agent is present in the stabilized non-blood-based sample at a concentration of between about 10mM and about 200mM (e.g., about 50mM, about 75mM, about 100mM, about 125mM, about 150mM, about 175mM, etc.). To further illustrate, when the non-blood-based sample comprises urine, a reducing agent is optionally present in the stabilized non-blood-based sample at a concentration of between about 10mM and about 20mM (e.g., about 12mM, about 14mM, about 16mM, about 18mM, etc.). Exemplary reducing agents utilized as described herein optionally comprise one or more of, e.g., dithiothreitol, dithioerithritol, 2-aminoethanethiol, 2-mercaptoethanol, or the like.

### Buffers

The stabilization component optionally further includes at least one buffer. Typically, the buffer is present in an amount that is sufficient to maintain a pH of the stabilized non-blood-based sample at about 6 or more. In some embodiments, the buffer is present in the stabilized non-blood-based sample at a concentration of between about 0.1mM and about 100mM (e.g., about 5mM, about 10mM, about 25mM, about 50 mM, about 75mM, etc.). For example, when the non-blood-based sample comprises urine, the buffer is optionally present in the stabilized non-blood-based sample at a concentration of between about 0.1mM and about 10mM (e.g., about 0.5mM, about 1mM, about 3mM, about 5mM, about 7mM, about 9mM, etc.). To further illustrate, the buffer optionally comprises one or more of, e.g., tris-(hydroxymethyl)aminoethane (TRIS), tris-(hydroxymethyl)aminomethane sodium phosphate, bis(2-hydroxyethyl)-imino-tris(hydroxymethyl)methane (BIS-TRIS), sodium acetate, sodium citrate, sodium tetraborate, glycine-sodium hydroxide, N-2-hydroxyethylpiperazine-N'-2-ethanesulfonic acid (HEPES), 3-[(1,1-dimethyl-hydroxyethyl)amino]-2-hydroxypropanesulfonic acid (AMPSO), 3-(cyclohexylamino)-2-hydroxy-1-propanesulfonic acid (CAPSO), 3-[N,N-bis(hydroxyethyl)amino]-2-hydroxypropanesulfonic acid (DIPSO), N-(2-hydroxyethyl)piperazine-N'-2-hydroxy propanesulfonic acid (HEPPSO), 3-(N-morpholino)-2-hydroxypropanesulfonicsulfonic acid (MOPSO), piperazine-N,N'-bis(2-hydroxypropanesulfonic acid) (POPSO), 3-[N-tris-(hydroxymethyl) methyl amino]-2-hydroxypropanesulfonic acid (TAPSO), 2-(N-morpholino)-ethanesulfonic acid (MES), 3-(N-morpholino)propanesulfonic acid (MOPS), piperazine-N,N'-bis(ethanesulfonic acid) (PIPES), acetate, citrate, hydrochloric acid, potassium chloride, glycine, potassium hydrogen phthalate, citric acid, acetic acid, sodium hydroxide, disodium hydrogen phthalate, sodium dihydrogen orthophosphate, dipotassium hydrogen phthalate, potassium dihydrogen orthophosphate, barbitone sodium, sodium tetraborate, sodium carbonate, sodium hydrogen carbonate, or the like.

### Detergents

In some embodiments, the stabilization component comprises at least one detergent. In these embodiments, the detergent typically comprises between about 0.001% and about 1% (e.g., about 0.01%, about 0.1%, about 0.5%, etc.) of a total weight of the stabilized non-blood-based sample. To further illustrate, when the non-blood-based sample comprises urine, the detergent optionally comprises between about 0.001% and about 0.01% (e.g., about 0.003%, about 0.005%, about 0.007%, about 0.009%, etc.) of a total weight of the stabilized non-blood-based sample. Optionally, the detergent comprises one or more of, e.g., polyoxyethylenesorbitan monolaurate (TWEEN® 20), sarcosine, sodium dodecyl sulfate, nonaethylene glycol octylphenol ether (TRITON® X-100), or the like.

### C. NUCLEIC ACID AMPLIFICATION REAGENTS

The reaction mixtures of the invention typically include stabilized non-blood based samples in which microbial nucleic acids are stabilized, as described herein. In addition, reaction mixtures also include nucleic acid amplification reagents. Exemplary nucleic acid amplification reagents include those that are useful in performing nucleic acid amplification reactions, such as primer nucleic acids, probe nucleic acids, nucleotide incorporating biocatalysts, extendible nucleotides, and the like. In some embodiments, for example, nucleic acid amplification reactions are performed utilizing these reaction mixtures to effect the detection of target microbial nucleic acids in samples, e.g., to aid in the diagnosis and/or prognosis of diseases. Nucleic acid amplification is also described further below.

Reaction mixtures are generally produced by combining stabilized non-blood based samples, or aliquots thereof, with quantities of the nucleic acid amplification reagents that are sufficient for performing the particular nucleic acid amplification method selected. The quantities of nucleic acid amplification reagents to be included in a given reaction mixture are well-known to persons of skill in the art in view of the selected nucleic acid amplification method. To illustrate, however, primer nucleic acids and extendible nucleotides (e.g., four dNTPs (dGTP, dCTP, dATP, dTTP)) are each present in a large molar excess in the reaction mixtures in certain embodiments. Probe and primer nucleic acids that can be utilized in the reaction mixtures of the invention are described further below. Suitable extendible nucleotides are readily available from many different commercial suppliers including, e.g., Roche Diagnostics Corporation (Indianapolis, IN, USA), Amersham Biosciences Corp. (Piscataway, NJ, USA), Applied Biosystems (Foster City, CA, USA), and the like.

In certain embodiments, stabilized non-blood based samples are subjected to one or more purification or other processing steps (e.g., to enrich for target microbial nucleic acids in the samples, etc.) prior to being combined with nucleic acid amplification reagents to form the reaction mixtures of the invention. Purification may involve techniques such as chemical extraction with salts, chloroform or phenol, sedimentation, centrifugation, chromatography or other techniques known to those of ordinary skill in the art. In other embodiments, reaction mixtures are produced without prior sample processing, e.g., to improve the throughput of the overall process relative to approaches that include sample processing steps.

The nucleotide incorporating biocatalysts utilized in the reaction mixtures and other aspect of the invention typically comprise enzymes, such as polymerases, terminal transferases, reverse transcriptases, telomerases, polynucleotide phosphorylases, and the like. In certain embodiments, for example, the enzyme includes a 5'-3' nuclease activity, a 3'-5' exonuclease activity, and/or is a thermostable enzyme. The enzyme is optionally derived from an organism, such as *Thermus antranikianii, Thermus aquaticus, Thermus caldophilus, Thermus chliarophilus, Thermus filiformis, Thermus flavus, Thermus igniterrae, Thermus lacteus, Thermus oshimai, Thermus ruber, Thermus rubens, Thermus scotoductus, Thermus silvanus, Thermus species* Z05, *Thermus species sps 17, Thermus thermophilus, Thermotoga maritima, Thermotoga neapolitana, Thermosipho africanus*, *Anaerocellum thermophilum, Bacillus caldotenax, Bacillus stearothermophilus,* or the like.

In some embodiments, the enzyme is modified. Exemplary modified enzymes include, e.g., G46E E678G CS5 DNA polymerases, G46E E678G CS6 DNA polymerases, Δ ZO5R polymerases, G46E L329A E678G CS5 DNA polymerases, and the like. Details relating to nucleotide incorporating biocatalysts are also provided in, e.g., U.S. Pat. No. 5,939,292, entitled "THERMOSTABLE DNA POLYMERASES HAVING REDUCED DISCRIMINATION AGAINST RIBO-NTPS," which issued August 17, 1999 to Gelfand et al., and U.S. Pat. Application Publication No. US 2002/0012970, entitled "HIGH TEMPERATURE REVERSE TRANSCRIPTION USING MUTANT DNA POLYMERASES," which published January 31, 2002 by Smith et al., and U.S. Patent Application No. 10/401,403, filed March 26, 2003, which are each incorporated by reference.

In certain embodiments, additional reagents are also added to the reaction mixtures of the invention. To illustrate, reaction mixtures also optionally include pyrophosphatases (e.g., a thermostable pyrophosphatase), e.g., for use in minimizing pyrophosphorolysis, uracil N-glycosylase (UNG) (e.g., a thermostable UNG), e.g., to protect against carry-over contamination, and the like.

### D. PREFERRED STABILIZED NON-BLOOD SAMPLES AND REACTION MIXTURES

Preferred non-blood-based samples of the present invention comprising:
- at least one non-blood-based sample that comprises microbial biomolecules; and,
- an effective amount of at least one stabilization component comprising at least one chaotropic reagent,
wherein the microbial biomolecules in the stabilized non-blood-based sample are stable at a temperature of about 20°C or more.

This non-blood-based sample preferably comprises one or more of: urine, seminal fluid, seminal plasma, prostatic fluid, vaginal fluid, vaginal scrapings, cervical fluid, uterine fluid, cervical scrapings, amniotic fluid, anal scrapings, feces, digestive fluid, nipple secretions, lymph, lung/bronchial washes, mucus, sputum, tears, buckle cells, saliva, tissue samples, spinal fluid, or perspiration.

Microbial biomolecules of this stabilized non-blood-based sample preferably comprise one or more of: nucleic acids, DNAs, RNAs, polypeptides, proteins, peptides, or prions.

Also, the microbial biomolecules of this stabilized non-blood-based sample preferably comprise one or more of: bacterial biomolecules, viral biomolecules, fungal biomolecules, or protozoal biomolecules and, even more preferably, comprise one or more of: *Neisseria gonorrhoeae* biomolecules, *Neisseria meningitidis* biomolecules, papillomaviral biomolecules, *Plasmodium falciparum* biomolecules, *Chlamydia muridarum* biomolecules, or *Chlamydia trachomatis* biomolecules.

The effective amount of the stabilization component of this stabilized non-blood-based sample preferably comprises about 45% or less of a total weight of the stabilized non-blood-based sample. Even more preferred this stabilized non-blood-based sample comprises urine and the effective amount of the stabilization component comprises about 15% or less of a total weight of the stabilized non-blood-based sample.

Preferably the stabilization component of this stabilized non-blood-based sample is substantially free of a chelating agent.

The chaotropic reagent of this stabilized non-blood-based sample preferably comprises one or more of: guanidine isothiocyanate or a salt thereof, guanidine hydrochloride or a salt thereof, urea, sodium iodide, potassium iodide, potassium chloride, ammonium thiocyanate, sodium thiocyanate, trifluoroethanol, or sodium perchlorate.

This stabilized non-blood-based sample preferably also comprise at least one transport medium.

The chaotropic reagent of this stabilized non-blood-based sample preferably is present in the stabilized non-blood-based sample at a concentration of about 5.0M or less. More preferably this stabilized non-blood-based sample comprises urine and the chaotropic reagent is present in the stabilized non-blood-based sample at a concentration of about 0.5M or less.

Preferably the levels of the microbial biomolecules in this stabilized non-blood-based sample deviate from one another by less than about 15% over at least about a seven day period whether a temperature of the stabilized non-blood-based sample is maintained at about 30°C or between about 8°C and about 2°C.

Preferably the stabilization component of this stabilized non-blood-based sample further comprises one or more of: an organic solvent, a reducing agent, a buffer, or a detergent as described below.

Preferably the stabilization component of this stabilized non-blood-based sample further comprises at least one organic solvent, which organic solvent more preferably comprises between about 0.1% and about 10% of a total weight of the stabilized non-blood-based sample. Even more preferabyl this stabilized non-blood-based sample comprises urine and the organic solvent comprises between about 0.1% and about 1% of a total weight of the stabilized non-blood-based sample. The organic solvent preferably comprises one or more of: polyethylene glycol, acetone, methanol, ethanol, or dimethylsulfoxide. In case this stabilized non-blood-based sample comprises polyethylene glycol it preferably comprises a molecular weight of between about 5000 daltons and about 15000 daltons.

Preferably the stabilization component of this stabilized non-blood-based sample further comprises at least one reducing agent, even more preferred this reducing agent comprises one or more of: dithiothreitol, dithioerithritol, 2-aminoethanethiol, or 2-mercaptoethanol. Preferably the reducing agent is present in the stabilized non-blood-based sample at a concentration of between about 10mM and about 200mM. Even more preferred the non-blood-based sample comprises urine and the reducing agent is present in the stabilized non-blood-based sample at a concentration of between about 10mM and about 20mM.

The stabilization component present in this stabilized non-blood-based sample further preferably comprises at least one buffer, which more preferably comprises one or more of: tris-(hydroxymethyl)aminoethane, tris-(hydroxymethyl)aminomethane sodium phosphate, bis(2-hydroxyethyl)-imino-tris(hydroxymethyl)methane, sodium acetate, sodium citrate, sodium tetraborate, glycine-sodium hydroxide, N-2-hydroxyethylpiperazine-N'-2-ethanesulfonic acid, 3- [(1,1-dimethyl-hydroxyethyl)amino] -2-hydroxypropanesulfonic acid, 3-(cyclohexylamino)-2-hydroxy-1-propanesulfonic acid, 3-[N,N-bis(hydroxyethyl)amino]-2-hydroxypropanesulfonic acid, N-(2-hydroxyethyl)piperazine-N'-2-hydroxy propanesulfonic acid, 3-(N-morpholino)-2-hydroxypropanesulfonicsulfonic acid, piperazine-N,N'-bis(2-hydroxypropanesulfonic acid), 3-[N-tris-(hydroxymethyl) methyl amino]-2-hydroxypropanesulfonic acid, 2-(N-morpholino)-ethanesulfonic acid, 3-(N-morpholino)propanesulfonic acid, piperazine-N,N'-bis(ethanesulfonic acid), acetate, citrate, hydrochloric acid, potassium chloride, glycine, potassium hydrogen phthalate, citric acid, acetic acid, sodium hydroxide, disodium hydrogen phthalate, sodium dihydrogen orthophosphate, dipotassium hydrogen phthalate, potassium dihydrogen orthophosphate, barbitone sodium, sodium tetraborate, sodium carbonate, or sodium hydrogen carbonate. The buffer preferably is present in this stabilized non-blood-based sample in an amount that is sufficient to maintain a pH of the stabilized non-blood-based sample at about 6 or more. More preferably the buffer is present in the stabilized non-blood-based sample at a concentration of between about 0.1mM and about 100mM, even more preferably at a concentration of between about 0.1mM and about 10mM.

In addition the stabilization component of this stabilized non-blood-based sample comprises at least one detergent, which preferably comprises one or more of: polyoxyethylenesorbitan monolaurate, sarcosine, sodium dodecyl sulfate, or nonaethylene glycol octylphenol ether. Preferably the detergent comprises between about 0.001% and about 1% of a total weight of the stabilized non-blood-based sample.

Even more preferred the non-blood-based sample comprises urine and the detergent comprises comprises between about 0.001% and about 0.01% of a total weight of the stabilized non-blood-based sample.

The present invention is also directed reacrtion mixtures. Preferably a reaction mixture of the present invention comprises:
- a stabilized non-blood based sample comprising:
   - at least one non-blood-based sample comprising one or more microbial nucleic acids; and
   - an effective amount of at least one stabilization component comprising at least one chaotropic reagent, wherein the stabilization component stabilizes the microbial nucleic acids at a temperature of about 20°C or more; and,
- at least one nucleic acid amplification reagent selected from the group consisting of: a primer nucleic acid, a probe nucleic acid, a nucleotide incorporating biocatalyst, and extendible nucleotides.

Preferably the non-blood-based sample ot this reaction mixture comprises one or more of: urine, seminal fluid, seminal plasma, prostatic fluid, vaginal fluid, vaginal scrapings, cervical fluid, uterine fluid, cervical scrapings, amniotic fluid, anal scrapings, feces, digestive fluid, nipple secretions, lymph, lung/bronchial washes, mucus, sputum, tears, buckle cells, saliva, tissue samples, spinal fluid, or perspiration.

The microbial nucleic acids present in this reaction mixture preferably comprise one or more of: *Neisseria gonorrhoeae* nucleic acids, *Neisseria meningitidis* nucleic acids, papillomaviral nucleic acids, *Plasmodium falciparum* nucleic acids, *Chlamydia muridarum* nucleic acids, or *Chlamydia trachomatis* nucleic acids.

Preferably the effective amount of the stabilization component present in this reaction mixture comprises about 45% or less of a total weight of the stabilized non-blood-based sample.

Preferably the stabilization component of this reaction mixture further comprises one or more of: an organic solvent, a reducing agent, a buffer, or a detergent.

Also, preferably the stabilization component of this reaction mixture is substantially free of a chelating agent.

### IV. METHODS OF BIOMOLECULE STABILIZATION AND ANALYSIS

The invention also provides methods of stabilizing microbial biomolecules in non-blood-based samples and for the analysis of those microbial biomolecules. The methods include contacting non-blood-based samples with effective amounts of stabilization components to produce stabilized non-blood-based samples. Stabilized non-blood-based samples, including sample types and sources, sample collection techniques, stabilized sample storage, are described in greater detail above and in the examples provided below. As referred to herein, the transport and/or storage of these samples (e.g., for subsequent analysis) is facilitated by these stabilization methods, especially since the samples do not need to be actively cooled (e.g., by refrigeration or the like) to effect biomolecule stabilization.

Typically, stabilized microbial biomolecules are subjected to one or more molecular analyses to provide, e.g., diagnostic information about patients from which the non-blood-based samples were derived. These analyses generally entail detecting the microbial biomolecules (e.g., proteins, nucleic acids, or the like) in the samples. In certain embodiments, microbial biomolecules are isolated (e.g., chromatographically, electrophoretically, etc.) from other components of stabilized non-blood-based samples, e.g., prior to being detected. In some embodiments, biomolecule detection includes amplifying detectable signals produced by the microbial biomolecules, and detecting those amplified signals. To further illustrate, when the target microbial biomolecules are nucleic acids, detection optionally includes amplifying segments of those nucleic acids, and detecting those amplified nucleic acids either concurrent with amplification (e.g., using a real-time PCR technique) or thereafter. Various illustrative automated and manual nucleic acid testing technologies are described further below.

In practicing the methods of the present invention, many conventional techniques in molecular biology are optionally utilized. These techniques are well known and are explained in, for example, Ausubel et al. (Eds.) Current Protocols in Molecular Biology, Volumes I, II, and III, (1997) (Ausubel 1), Ausubel et al. (Eds.), Short Protocols in Molecular Biology: A Compendium of Methods from Current Protocols in Molecular Biology, 5^{th} Ed., John Wiley & Sons, Inc. (2002) (Ausubel 2), Sambrook et al., Molecular Cloning: A Laboratory Manual, 3rd Ed., Cold Spring Harbor Laboratory Press (2000) (Sambrook), Innis et al. (Eds.) PCR Protocols: A Guide to Methods and Applications, Elsevier Science & Technology Books (1990) (Innis), Berger and Kimmel, Guide to Molecular Cloning Techniques: Methods in Enzymology, Volume 152, Academic Press, Inc. (Berger), Vorbruggen et al., Handbook of Nucleoside Synthesis, Organic Reactions Series, #60, John Wiley & Sons, Inc. (2001), Gait (Ed.) Oligonucleotide Synthesis, Oxford University Press (1984), Hames and Higgins, Nucleic Acid Hybridization, Practical Approach Series, Oxford University Press (1997), and Hames and Higgins (Eds.) Transcription and Translation, Practical Approach Series, Oxford University Press (1984), all of which are incorporated by reference.

### A. NUCLEIC ACID ANALYSIS TECHNOLOGIES

Examples of general types of nucleic acid analysis technologies that can be used or adapted for use to analyze microbial nucleic acids in or from the reactions mixtures of the invention include nucleic acid probe-based techniques, signal amplification assays, and nucleic acid amplification tests. Nucleic acid probe-based assays typically include the use of labeled oligonucleotide probes that hybridize to target nucleic acids in the sample. Certain signal amplification assays are essentially nucleic acid probe-based tests that use multiple labels to amplify the signal of the assay. Examples of these types of tests include bDNA assays (Bayer Diagnostics, Inc., Emeryville, CA, USA) and the Hybrid Capture assay (Digene Corp., Gaithersburg, MD, USA). A common characteristic among nucleic acid amplification tests is that they are typically designed to amplify nucleic acid sequences that are specific for the organism being detected. Nucleic acid amplification tests generally have greater sensitivity than the other approaches to nucleic acid analysis. This improved sensitivity is typically attributable to their ability to produce a positive signal from as little as a single copy of the target nucleic acid. Various commercial nucleic acid amplification tests that are optionally adapted for use with the stabilized non-blood-based samples and methods of the invention generally differ in their amplification methods and their target nucleic acid sequences. Examples of these commercial tests include the AMPLICOR® and COBAS AMPLICOR® assays (Roche Diagnostics Corporation, Indianapolis, IN, USA), which use polymerase chain reactions (PCR); the LCx® test (Abbott Laboratories, Abbott Park, IL, USA), which uses ligase chain reactions (LCR); the BDProbeTec™ ET test (Becton, Dickinson and Company, Franklin Lakes, N.J., USA), which uses strand displacement amplification (SDA); and the APTIMA™ assay (Gen-Probe, Inc., San Diego, CA, USA), which uses transcription-mediated amplification (TMA). Nucleic acid amplification is described further below.

Other nucleic acid analytical techniques that can be adapted for use with the stabilized non-blood-based samples and methods of the invention include genetic transformation testing (GTT). This technique is also described in, e.g., Jaffe et al., "Diagnosis of gonorrhea using a genetic transformation test on mailed clinical specimens," J Infect Dis. 146(2):275-279 (1982), which is incorporated by reference.

Oligonucleotide probes and/or primers that can be used as microbial biomolecule detection reagents to detect microbial biomolecules in or from the stabilized non-blood-based samples of the invention are optionally prepared using essentially any technique known in the art. In certain embodiments, for example, oligonucleotide probes and/or primers are synthesized chemically using various nucleic acid synthesis methods including, e.g., the solid phase phosphoramidite triester method described by Beaucage and Caruthers Tetrahedron Letts. 22(20):1859-1862 (1981), which is incorporated by reference, or another synthesis technique known in the art, e.g., using an automated synthesizer, as described in Needham-VanDevanter et al. Nucleic Acids Res. 12:6159-6168 (1984), which is incorporated by reference. A wide variety of equipment is commercially available for automated oligonucleotide synthesis. Multi-nucleotide synthesis approaches (e.g., tri-nucleotide synthesis, etc.) are also optionally utilized. Moreover, oligonucleotides are optionally produced with various modifications. In certain embodiments, for example, primers include restriction site linkers, e.g., to facilitate subsequent amplicon cloning or the like. To further illustrate, primers are also optionally modified to improve the specificity of amplification reactions as described in, e.g., U.S. Pat. No. 6,001,611, entitled "MODIFIED NUCLEIC ACID AMPLIFICATION PRIMERS," issued December 14, 1999 to Will, which is incorporated by reference. Primers and probes can also be synthesized with various other modifications known in the art.

Probes and/or primers utilized in the methods of the invention are optionally labeled to permit detection of probe-target hybridization duplexes. In general, a label can be any moiety that can be attached to a nucleic acid or other microbial biomolecule detection reagent and provide a detectable signal (e.g., a quantifiable signal). Labels may be attached to microbial biomolecule detection reagents directly or indirectly by a variety of techniques known in the art. To illustrate, depending on the type of label used, the label can be attached to a terminal (5' or 3' end of an oligonucleotide primer and/or probe) or a non-terminal nucleotide, and can be attached indirectly through linkers or spacer arms of various sizes and compositions. Using commercially available phosphoramidite reagents, one can produce oligomers containing functional groups (e.g., thiols or primary amines) at either the 5' or 3' terminus via an appropriately protected phosphoramidite, and can label such oligonucleotides using protocols described in, for example, Innis, *supra.*

Essentially any label is optionally utilized to label the microbial biomolecule detection reagents (e.g., oligonucleotide primers and probes, antibodies, etc.) described herein. In some embodiments, for example, the label comprises a fluorescent dye (e.g., a rhodamine dye (e.g., R6G, R110, TAMRA, ROX, etc.), a fluorescein dye (e.g., JOE, VIC, TET, HEX, FAM, etc.), a halofluorescein dye, a cyanine dye (e.g., CY3, CY3.5, CY5, CY5.5, etc.), a BODIPY® dye (e.g., FL, 530/550, TR, TMR, etc.), an ALEXA FLUOR® dye (e.g., 488, 532, 546, 568, 594, 555, 653, 647, 660, 680, etc.), a dichlororhodamine dye, an energy transfer dye (e.g., BIGDYE™ v 1 dyes, BIGDYE™ v 2 dyes, BIGDYE™ v 3 dyes, etc.), Lucifer dyes (e.g., Lucifer yellow, etc.), CASCADE BLUE® , Oregon Green, and the like. Additional examples of fluorescent dyes are provided in, e.g., Haugland, Molecular Probes Handbook of Fluorescent Probes and Research Products, Ninth Ed. (2003) and the updates thereto, which are each incorporated by reference. Fluorescent dyes are generally readily available from various commercial suppliers including, e.g., Molecular Probes, Inc. (Eugene, OR), Amersham Biosciences Corp. (Piscataway, NJ), Applied Biosystems (Foster City, CA), etc. Other labels include, e.g., biotin, weakly fluorescent labels (Yin et al. (2003) Appl Environ Microbiol. 69(7):3938, Babendure et al. (2003) Anal. Biochem. 317(1):1, and Jankowiak et al. (2003) Chem Res Toxicol. 16(3):304), non-fluorescent labels, colorimetric labels, chemiluminescent labels (Wilson et al. (2003) Analyst. 128(5):480 and Roda et al. (2003) Luminescence 18(2):72), Raman labels, electrochemical labels, bioluminescent labels (Kitayama et al. (2003) Photochem Photobiol. 77(3):333, Arakawa et al. (2003) Anal. Biochem. 314(2):206, and Maeda (2003) J. Pharm. Biomed. Anal. 30(6):1725), and an alpha-methyl-PEG labeling reagent as described in, e.g., U.S. Provisional Patent Application No. 60/428,484, filed on Nov. 22, 2002, which references are each incorporated by reference.

In addition, essentially any nucleic acid (and virtually any labeled nucleic acid, whether standard or non-standard) can be custom or standard ordered from any of a variety of commercial sources, such as The Midland Certified Reagent Company, The Great American Gene Company, ExpressGen Inc., Operon Technologies Inc., Proligo LLC, and many others.

Probes and primers for detecting N. *gonorrhoeae* and C. *trachomatis* nucleic acids, which are optionally utilized to perform certain embodiments of the methods of the invention, are described in, e.g., U.S. Pat. No. 5,550,040 to Purohit et al., and U.S. Pat. No. 6,090,557 to Weiss, which are both incorporated by reference. Other probes and/or primers that detectably bind to other target microbes are known in the art or can be readily designed by persons of skill in the art.

In certain embodiments, oligonucleotide probes designed to hybridize with target microbial biomolecules are covalently or noncovalently attached to solid supports. In these embodiments, labeled amplicons derived from microbial nucleic acids in the stabilized non-blood-based samples of the invention are typically contacted with these solid support-bound probes to effect hybridization and detection. In other embodiments, amplicons are attached to solid supports and contacted with labeled probes. In still other embodiments, nucleic acids are detected free in solution.

Essentially any substrate material is optionally adapted for use as a solid support. In certain embodiments, for example, substrates are fabricated from silicon, glass, or polymeric materials (e.g., glass or polymeric microscope slides, silicon wafers, wells of microwell plates, etc.). Suitable glass or polymeric substrates, including microscope slides, are available from various commercial suppliers, such as Fisher Scientific (Pittsburgh, PA) or the like. In some embodiments, solid supports utilized in the invention are membranes. Suitable membrane materials are optionally selected from, e.g. polyaramide membranes, polycarbonate membranes, porous plastic matrix membranes (e.g., POREX® Porous Plastic, etc.), nylon membranes, ceramic membranes, polyester membranes, polytetrafluoroethylene (TEFLON® ) membranes, nitrocellulose membranes, or the like. Many of these membranous materials are widely available from various commercial suppliers, such as, P.J. Cobert Associates, Inc. (St. Louis, MO, USA), Millipore Corporation (Bedford, MA, USA), or the like. Other exemplary solid supports that are optionally utilized include, e.g., ceramics, metals, resins, gels, plates, beads (e.g., magnetic microbeads, etc.), whiskers, fibers, combs, single crystals, self-assembling monolayers, and the like.

Nucleic acids are directly or indirectly (e.g., via linkers, such as bovine serum albumin (BSA) or the like) attached to the supports, e.g., by any available chemical or physical method. A wide variety of linking chemistries are available for linking molecules to a wide variety of solid supports. More specifically, nucleic acids may be attached to the solid support by covalent binding, such as by conjugation with a coupling agent or by non-covalent binding, such as electrostatic interactions, hydrogen bonds or antibody-antigen coupling, or by combinations thereof. Typical coupling agents include biotin/avidin, biotin/streptavidin, *Staphylococcus aureus* protein A/IgG antibody F_{c} fragment, and streptavidin/protein A chimeras (Sano et al. (1991) Bio/Technology 9:1378, which is incorporated by reference), or derivatives or combinations of these agents. Nucleic acids may be attached to the solid support by a photocleavable bond, an electrostatic bond, a disulfide bond, a peptide bond, a diester bond or a combination of these bonds. Nucleic acids are also optionally attached to solid supports by a selectively releasable bond such as 4,4'-dimethoxytrityl or its derivative.

Cleavable attachments can be created by attaching cleavable chemical moieties between the probes and the solid support including, e.g., an oligopeptide, oligonucleotide, oligopolyamide, oligoacrylamide, oligoethylene glycerol, alkyl chains of between about 6 to 20 carbon atoms, and combinations thereof. These moieties may be cleaved with, e.g., added chemical agents, electromagnetic radiation, or enzymes. Exemplary attachments cleavable by enzymes include peptide bonds, which can be cleaved by proteases, and phosphodiester bonds which can be cleaved by nucleases.

Chemical agents such as β-mercaptoethanol, dithiothreitol (DTT) and other reducing agents cleave disulfide bonds. Other agents which may be useful include oxidizing agents, hydrating agents and other selectively active compounds. Electromagnetic radiation such as ultraviolet, infrared and visible light cleave photocleavable bonds. Attachments may also be reversible, e.g., using heat or enzymatic treatment, or reversible chemical or magnetic attachments. Release and reattachment can be performed using, e.g., magnetic or electrical fields.

A number of array systems have been described and can be adapted for use in the detection of target microbial nucleic acids. Aspects of array construction and use are also described in, e.g., Sapolsky et al. (1999) "High-throughput polymorphism screening and genotyping with high-density oligonucleotide arrays" Genetic Analysis: Biomolecular Engineering 14:187-192, Lockhart (1998) "Mutant yeast on drugs" Nature Medicine 4:1235-1236, Fodor (1997) "Genes, Chips and the Human Genome" FASEB Journal 11:A879, Fodor (1997) "Massively Parallel Genomics" Science 277: 393-395, and Chee et al. (1996) "Accessing Genetic Information with High-Density DNA Arrays" Science 274:610-614, all of which are incorporated by reference.

Amplification methods that are optionally utilized to detect microbial biomolecules in or from the stabilized non-blood-based samples of the invention include, e.g., various polymerase, ligase, or reverse-transcriptase mediated amplification methods, such as the polymerase chain reaction (PCR), the ligase chain reaction (LCR), reverse-transcription PCR (RT-PCR), and/or the like. Details regarding the use of these and other amplification methods can be found in any of a variety of standard texts, including, e.g., Berger, Sambrook, Ausubel 1 and 2, and Innis, which are referred to above. Many available biology texts also have extended discussions regarding PCR and related amplification methods. Nucleic acid amplification is also described in, e.g., Mullis et al., (1987) U.S. Patent No. 4,683,202 and Sooknanan and Malek (1995) Biotechnology 13:563, which are both incorporated by reference. Improved methods of amplifying large nucleic acids by PCR are summarized in Cheng et al. (1994) Nature 369:684, which is incorporated by reference. In certain embodiments, duplex PCR is utilized to amplify target nucleic acids. Duplex PCR amplification is described further in, e.g., Gabriel et al. (2003) "Identification of human remains by immobilized sequence-specific oligonucleotide probe analysis of mtDNA hypervariable regions I and II," Croat. Med. J. 44(3)293 and La et al. (2003) "Development of a duplex PCR assay for detection of Brachyspira hyodysenteriae and Brachyspira pilosicoli in pig feces," J. Clin. Microbiol. 41(7):3372, which are both incorporated by reference. Optionally, labeled primers (e.g., biotinylated primers, etc.) are utilized to amplify nucleic acids in a sample, e.g., to facilitate detection of hybridization between amplicons and the oligonucleotide probes of the invention.

Amplicons are optionally recovered and purified from other reaction components by any of a number of methods well known in the art, including electrophoresis, chromatography, precipitation, dialysis, filtration, and/or centrifugation. Aspects of nucleic acid purification are described in, e.g., Douglas et al., DNA Chromatography, Wiley, John & Sons, Inc. (2002), and Schott, Affinity Chromatography: Template Chromatography of Nucleic Acids and Proteins, Chromatographic Science Series, #27, Marcel Dekker (1984), both of which are incorporated by reference. In certain embodiments, amplicons are not purified prior to detection, such as when amplicons are detected simultaneous with amplification. The detection of amplicons is described further below.

Hybridization of oligonucleotide probes to target microbial nucleic acids, such as N. *gonorrhoeae* and/or C. *trachomatis* nucleic acids, can be accomplished by choosing appropriate hybridization conditions. The stability of the probe:target nucleic acid hybrid is typically selected to be compatible with the assay and washing conditions so that stable, detectable hybrids form only between the probes and target microbial nucleic acids. Manipulation of one or more of the different assay parameters determines the exact sensitivity and specificity of a particular hybridization assay.

More specifically, hybridization between complementary bases of DNA, RNA, PNA, or combinations of DNA, RNA and PNA, occurs under a wide variety of conditions that vary in temperature, salt concentration, electrostatic strength, buffer composition, and the like. Examples of these conditions and methods for applying them are described in, e.g., Tijssen, Hybridization with Nucleic Acid Probes, Vol. 24, Elsevier Science (1993), and Hames and Higgins, *supra,* which are both incorporated by reference. Hybridization generally takes place between about 0°C and about 70°C, for periods of from about one minute to about one hour, depending on the nature of the sequence to be hybridized and its length. However, it is recognized that hybridizations can occur in seconds or hours, depending on the conditions of the reaction. To illustrate, typical hybridization conditions for a mixture of two 20-mers is to bring the mixture to 68°C, followed by cooling to room temperature (22°C) for five minutes or at very low temperatures such as 2°C in 2 microliters. Hybridization between nucleic acids may be facilitated using buffers such as Tris-EDTA (TE), Tris-HCl and HEPES, salt solutions (e.g. NaCl, KCl, CaCl₂), or other aqueous solutions, reagents and chemicals. Examples of these reagents include single-stranded binding proteins such as Rec A protein, T4 gene 32 protein, *E. coli* single-stranded binding protein and major or minor nucleic acid groove binding proteins. Other examples of such reagents and chemicals include divalent ions, polyvalent ions and intercalating substances such as ethidium bromide, actinomycin D, psoralen, and angelicin. An exemplary hybridization procedure of use in the present invention follows similar conditions as specified in the COBAS AMPLICOR® Chlamydia trachomatis (CT)/ Neisseria gonorrhoeae (NG) Test protocol (Roche Diagnostics Corporation, Indianapolis, IN).

Any available method for detecting target microbial biomolecules and/or amplicons thereof can be used in the present invention. Common approaches include real time amplification detection with 5'-nuclease probes or molecular beacons, detection of intercalating dyes, detection of labels incorporated into the amplification probes or the amplified nucleic acids themselves, e.g., following electrophoretic separation of the amplification products from unincorporated label, hybridization based assays (e.g., array based assays), and/or detection of secondary reagents that bind to the nucleic acids. For example, a 5'-nuclease probe or a molecular beacon is optionally designed to include a oligonucleotide sequence that targets a particular microbial nucleic acid (e.g., a nucleic acid from *Neisseria gonorrhoeae, Neisseria meningitidis,* papilloma virus, *Plasmodium falciparum, Chlamydia muridarum, Chlamydia trachomatis,* etc.). Molecular beacons or 5'-nuclease probes are described further below. These general approaches are also described in, e.g., Sambrook, and Ausubel 1 and 2.

In certain embodiments, real time PCR assay systems that include one or more 5'-nuclease probes are used for detecting amplified target microbial nucleic acids. These systems operate by using the endogenous endonuclease activity of certain polymerases to cleave a quencher or label free from an oligonucleotide that comprises the quencher and label, resulting in unquenching of the label. The polymerase only cleaves the quencher or label upon initiation of replication, i.e., when the oligonucleotide is bound to the template and the polymerase extends the primer. Thus, an appropriately labeled probe nucleic acid and a polymerase comprising the appropriate nuclease activity can be used to detect a target microbial nucleic acid of interest. Real time PCR product analysis by, e.g., FRET or the like (and related real time reverse-transcription PCR) provides a well-known technique for real time PCR monitoring that has been used in a variety of contexts, which can be adapted for use with the methods described herein *(see,* Laurendeau et al. (1999) "TaqMan PCR-based gene dosage assay for predictive testing in individuals from a cancer family with INK4 locus haploinsufficiency" Clin Chem 45(7):982-6; Laurendeau et al. (1999) "Quantitation of MYC gene expression in sporadic breast tumors with a real-time reverse transcription-PCR assay" Clin Chem 59(12):2759-65; and Kreuzer et al. (1999) "LightCycler technology for the quantitation of bcr/ab1 fusion transcripts" Cancer Research 59(13):3171-4, all of which are incorporated by reference).

Exemplary commercially available systems that are optionally utilized to detect target microbial biomolecules using the nucleic acid detection reagents described herein include, e.g., a COBAS AMPLICOR® Analyzer, which is available from Roche Diagnostics Corporation (Indianapolis, IN, USA), a LUMINEX 100™ system, which is available from the Luminex Corporation (Austin, TX, USA), and the like.

Molecular beacons are oligonucleotides designed for real time detection and quantification of target nucleic acids. The 5' and 3' termini of molecular beacons collectively comprise a pair of moieties, which confers the detectable properties of the molecular beacon. One of the termini is attached to a fluorophore and the other is attached to a quencher molecule capable of quenching a fluorescent emission of the fluorophore. To illustrate, one example fluorophore-quencher pair can use a fluorophore, such as EDANS or fluorescein, e.g., on the 5'-end and a quencher, such as Dabcyl, e.g., on the 3'-end. When the molecular beacon is present free in solution, i.e., not hybridized to a second nucleic acid, the stem of the molecular beacon is stabilized by complementary base pairing. This self-complementary pairing results in a "hairpin loop" structure for the molecular beacon in which the fluorophore and the quenching moieties are proximal to one another. In this confirmation, the fluorescent moiety is quenched by the quenching moiety. The loop of the molecular beacon typically comprises the oligonucleotide probe and is accordingly complementary to a sequence to be detected in the target microbial nucleic acid, such that hybridization of the loop to its complementary sequence in the target forces disassociation of the stem, thereby distancing the fluorophore and quencher from each other. This results in unquenching of the fluorophore, causing an increase in fluorescence of the molecular beacon.

Details regarding standard methods of making and using molecular beacons are well established in the literature and molecular beacons are available from a number of commercial reagent sources. Further details regarding methods of molecular beacon manufacture and use are found, e.g., in Leone et al. (1995) "Molecular beacon probes combined with amplification by NASBA enable homogenous real-time detection of RNA," Nucleic Acids Res. 26:2150-2155; Kostrikis et al. (1998) "Molecular beacons: spectral genotyping of human alleles" Science 279:1228-1229; Fang et al. (1999) "Designing a novel molecular beacon for surface-immobilized DNA hybridization studies" J. Am. Chem. Soc. 121:2921-2922; and Marras et al. (1999) "Multiplex detection of single-nucleotide variation using molecular beacons" Genet. Anal. Biomol. Eng. 14:151-156, all of which are incorporated by reference. A variety of commercial suppliers produce standard and custom molecular beacons, including Oswel Research Products Ltd. (UK), Research Genetics (a division of Invitrogen, Huntsville, AL, USA), the Midland Certified Reagent Company (Midland, TX, USA), and Gorilla Genomics, LLC (Alameda, CA, USA). A variety of kits which utilize molecular beacons are also commercially available, such as the Sentinel™ Molecular Beacon Allelic Discrimination Kits from Stratagene (La Jolla, CA, USA) and various kits from Eurogentec SA (Belgium) and Isogen Bioscience BV (Netherlands).

Detectors are typically structured to detect detectable signals produced, e.g., in or proximal to another component of the given assay system (e.g., in container, on a solid support, etc.). Suitable signal detectors that are optionally utilized, or adapted for use, herein detect, e.g., fluorescence, phosphorescence, radioactivity, absorbance, refractive index, luminescence, mass, or the like. Detectors optionally monitor one or a plurality of signals from upstream and/or downstream of the performance of, e.g., a given assay step. For example, detectors optionally monitor a plurality of optical signals, which correspond in position to "real time" results. Example detectors or sensors include photomultiplier tubes, CCD arrays, optical sensors, temperature sensors, pressure sensors, pH sensors, conductivity sensors, scanning detectors, or the like. More specific exemplary detectors that are optionally utilized in performing the methods of the invention include, e.g., resonance light scattering detectors, emission spectroscopes, fluorescence spectroscopes, phosphorescence spectroscopes, luminescence spectroscopes, spectrophotometers, photometers, and the like. Detectors are also described in, e.g., Skoog et al., Principles of Instrumental Analysis, 5^{th} Ed., Harcourt Brace College Publishers (1998) and Currell, Analytical Instrumentation: Performance Characteristics and Quality, John Wiley & Sons, Inc. (2000), both of which are incorporated by reference.

### B. PROTEIN ANALYSIS TECHNOLOGIES

Microbial proteins that have been preserved in the stabilized non-blood-based samples of the invention can also be detected, e.g., to assist in providing a diagnosis for a patient. Essentially any available technique for the detection of proteins is optionally utilized in the methods of the invention. Exemplary protein analysis technologies include, e.g., one- and two-dimensional SDS-PAGE-based separation and detection, immunoassays (e.g., western blotting, etc.), aptamer-based detection, mass spectrometric detection, and the like. These and other techniques are generally well-known in the art.

To illustrate, antibodies suitable for use in immunoassays may be prepared by conventional methodology and/or by genetic engineering. Antibody fragments may be genetically engineered, e.g., from the variable regions of the light and/or heavy chains (V_{H} and V_{L}), including the hypervariable regions, or from both the V_{H} and V_{L} regions. For example, the term "antibodies" as used herein includes polyclonal and monoclonal antibodies and biologically active fragments thereof including among other possibilities "univalent" antibodies (Glennie et al. (1982) Nature 295:712); Fab proteins including Fab' and F(ab')₂ fragments whether covalently or non-covalently aggregated; light or heavy chains alone, typically variable heavy and light chain regions (V_{H} and V_{L} regions), and more typically including the hypervariable regions (otherwise known as the complementarity determining regions (CDRs) of the V_{H} and V_{L} regions); F_{c} proteins; "hybrid" antibodies capable of binding more than one antigen; constant-variable region chimeras; "composite" immunoglobulins with heavy and light chains of different origins; "altered" antibodies with improved specificity and other characteristics as prepared by standard recombinant techniques, by mutagenic techniques, or other directed evolutionary techniques known in the art.

The antibodies utilized in the methods of the invention may be labeled or unlabeled. Suitable labels include, e.g., radionuclides, enzymes, coenzymes, fluorescent dyes, chemiluminescent dyes, chromogens, enzyme substrates or co-factors, enzyme inhibitors, free radicals, and the like. Such labeled reagents may be used in a variety of well known assays, such as radioimmunoassays, enzyme immunoassays, e.g., ELISA, fluorescent immunoassays, and the like. See, e.g., U.S. Pat. Nos. 3,766,162; 3,791,932; 3,817,837; and 4,233,402, which are each incorporated by reference. Additional labels are described further herein.

Microbial proteins can also be detected using aptamers including photoaptamers. Aptamers generally are single-stranded oligonucleotides (e.g., typically DNA for diagnostic applications) that assume a specific, sequence-dependent shape and binds to target proteins based on a "lock-and-key" fit between the two molecules. Aptamers can be identified using the SELEX process (Gold (1996) "The SELEX process: a surprising source of therapeutic and diagnostic compounds," Harvey Lect. 91:47-57, which is incorporated by reference). Aptamer arrays are commercially available from various suppliers including, e.g., SomaLogic, Inc. (Boulder, CO, USA).

The detection of proteins via mass includes various formats that can be adapted for use in the methods of the invention. Exemplary formats include matrix assisted laser desorption/ionization- (MALDI) and surface enhanced laser desorption/ionization-based (SELDI) detection. MALDI- and SELDI-based detection are also described in, e.g., Weinberger et al. (2000) "Recent trends in protein biochip technology," Pharmacogenomics 1(4):395-416, Forde et al. (2002) "Characterization of transcription factors by mass spectrometry and the role of SELDI-MS," Mass Spectrom. Rev. 21(6):419-439, and Leushner (2001) "MALDI TOF mass spectrometry: an emerging platform for genomics and diagnostics," Expert Rev. Mol. Diagn. 1(1):11-18, which are each incorporated by reference. Protein chips and related instrumentation are available from commercial suppliers, such as Ciphergen Biosystems, Inc. (Fremont, CA, USA).

### C PREFERRED METHODS OF BIOMOLECULE STABILIZATION AND ANALYSIS

The present invention is also directed to methods for biomolecule stabilization and analysis. A preferred method of the present invention is a method of stabilizing microbial biomolecules in non-blood-based samples, the method comprising:
- providing at least one non-blood-based sample comprising the microbial biomolecules; and,
- contacting the non-blood-based sample with an effective amount of at least one stabilization component comprising at least one chaotropic reagent to produce a stabilized non-blood-based sample, wherein the stabilization component stabilizes the microbial biomolecules at a temperature of about 20°C or more.

Preferably the microbial biomolecules in this method comprise one or more of: nucleic acids, DNAs, RNAs, polypeptides, proteins, peptides, or prions.

Also preferably the stabilization component used in this method is substantially free of a chelating agent.

Preferably the effective amount of the stabilization component used in this method comprises about 45% or less of a total weight of the stabilized non-blood-based sample. More preferrably the non-blood-based sample comprises urine and the effective amount of the stabilization component used in this method comprises about 15% or less of a total weight of the stabilized non-blood-based sample.

Preferably the chaotropic reagent used in this method comprises one or more of: guanidine isothiocyanate or a salt thereof, guanidine hydrochloride or a salt thereof, urea, sodium iodide, potassium iodide, potassium chloride, ammonium thiocyanate, sodium thiocyanate, trifluoroethanol, or sodium perchlorate.

This method preferably further comprises contacting the non-blood-based sample with at least one transport medium.

Preferably the levels of the microbial biomolecules in the stabilized non-blood-based sample of this method deviate from one another by less than about 15% over at least about a seven day period whether a temperature of the stabilized non-blood-based sample is maintained at about 30°C or between about 8°C and about 2°C.

This method preferably further comprises:
- isolating one or more of the microbial biomolecules after the contacting step.

This method more preferably further comprises:
- detecting one or more of the microbial biomolecules.

Preferably this detection step comprises:
- amplifying a detectable signal produced by the microbial biomolecules, or by a microbial biomolecule detection reagent that detectably binds to at least one of the microbial biomolecules, to produce an amplified signal; and,
- detecting the amplified signal.

More preferably the microbial biomolecules comprise nucleic acids and the detecting step of this method comprises:
- amplifying at least a segment of one or more of the nucleic acids to produce amplified nucleic acids; and,
- detecting one or more of the amplified nucleic acids during or after the amplifying step.

Preferably the stabilization component used in this method further comprises one or more of: an organic solvent, a reducing agent, a buffer, or a detergent as described below.

Preferably the stabilization component used in this method further comprises at least one organic solvent, which preferably comprises one or more of: polyethylene glycol, acetone, or dimethylsulfoxide.

Also preferably the stabilization component used in this method further comprises at least one reducing agent, which preferably comprises one or more of: dithiothreitol, 2-aminoethanethiol, or 2-mercaptoethanol.

The stabilization component used in this method preferably further comprises at least one buffer, which preferably comprises one or more of: tris-(hydroxymethyl)aminoethane, tris-(hydroxymethyl)aminomethane sodium phosphate, bis(2-hydroxyethyl)-imino-tris(hydroxymethyl)methane, sodium acetate, sodium citrate, sodium tetraborate, glycine-sodium hydroxide, N-2-hydroxyethylpiperazine-N'-2-ethanesulfonic acid, 3-[(1,1-dimethyl-hydroxyethyl)amino]-2-hydroxypropanesulfonic acid, 3-(cyclohexylamino)-2-hydroxy-1-propanesulfonic acid, 3-[N,N-bis(hydroxyethyl)amino]-2-hydroxypropanesulfonic acid, N-(2-hydroxyethyl)piperazine-N'-2-hydroxy propanesulfonic acid, 3-(N-morpholino)-2-hydroxypropanesulfonicsulfonic acid, piperazine-N,N'-bis(2-hydroxypropanesulfonic acid), 3-[N-tris-(hydroxymethyl) methyl amino]-2-hydroxypropanesulfonic acid, 2-(N-morpholino)-ethanesulfonic acid, 3-(N-morpholino)propanesulfonic acid, piperazine-N,N'-bis(ethanesulfonic acid), acetate, citrate, hydrochloric acid, potassium chloride, glycine, potassium hydrogen phthalate, citric acid, acetic acid, sodium hydroxide, disodium hydrogen phthalate, sodium dihydrogen orthophosphate, dipotassium hydrogen phthalate, potassium dihydrogen orthophosphate, barbitone sodium, sodium tetraborate, sodium carbonate, or sodium hydrogen carbonate.

In addition, the stabilization component used in this method preferably further comprises at least one detergent, which preferably comprises one or more of: polyoxyethylenesorbitan monolaurate, sarcosine, sodium dodecyl sulfate, or α-[4-(1,1,3,3-tetramethylbutyl)phenyl]-omega-hydroxypoly(oxy-1,2-ethanediyl).

Finally a further subject of the present invention is the stabilized non-blood-based sample produced by the above described method.

### V. KITS

The invention also provides kits for the stabilization of microbial biomolecules in non-blood-based samples. These kits typically include at least one stabilization component as described herein and instructions for contacting non-blood-based samples with effective amounts of the stabilization components to produce stabilized non-blood-based samples in which microbial biomolecules are stable at temperatures of about 20°C or more. In certain embodiments, stabilization components comprise a stabilization solution, whereas in others, stabilization components include one or more solid materials (e.g., lyophilized or powdered chaotropic reagents or other compounds). Generally, chaotropic reagents are present in the stabilization components (e.g., stabilization solutions) at concentrations of about 5.0M or less (e.g., about 4.5M, about 4.0M, about 3.5M, etc.). In some embodiments, stabilization components further include organic solvents. Typically, an organic solvent comprises about 50% or less (e.g., about 40%, about 30%, about 20%, about 10%, etc.) of a total weight of the particular stabilization component. Optionally, stabilization components further comprise reducing agents. In these embodiments, reducing agents are generally present in the stabilization components at concentrations of about 200mM or less (e.g., about 150mM, 100mM, 50mM, etc.). In some embodiments, stabilization components further comprise buffers, which are typically present in the stabilization components at concentrations of about 100mM or less (e.g., about 75mM, about 50mM, about 25mM, etc.). In certain embodiments, stabilization components further include detergents. In some of these embodiments, detergents comprise about 1% or less (e.g., about 0.8%, about 0.6%, about 0.4%, about 0.2%, etc.) of a total weight of the particular stabilization component. Stabilization components, or compounds thereof, are generally packaged in one or more containers. In some of these embodiments, the kits further include at least one transport medium, e.g., disposed in containers (e.g., specimen containers, etc.).

Optionally, kits further include instructions for detecting the microbial biomolecules in the stabilized non-blood-based sample. In these embodiments, for example, the kits may also include suitably packaged reagents (e.g., oligonucleotide probes and/or primers, or other microbial biomolecule detection reagents) and materials needed, e.g., for nucleic acid immobilization, hybridization, and detection, such solid supports, buffers, enzymes, and nucleic acid standards. Optionally, the microbial biomolecule detection reagents (e.g., oligonucleotide probes, antibodies, etc.) of the invention are provided already attached or otherwise immobilized on solid supports. As another option, microbial biomolecule detection reagents are provided free in solution in containers, e.g., for performing the detection methods of the invention in the solution phase. In some of these embodiments, microbial biomolecule detection reagents of the kits comprise labels and/or quencher moieties, e.g., when microbial biomolecule detection reagents are in the form of molecular beacons, 5'-nuclease probes, or the like. In certain embodiments, kits further include labeled primers for amplifying target microbial nucleic acids.

Kits optionally also include one or more of: a set of instructions for contacting the microbial biomolecule detection reagents with nucleic acids from a sample or amplicons thereof and detecting binding between the microbial biomolecule detection reagents and target microbial biomolecules (e.g., *N*. *gonorrhoeae* and/or *C*. *trachomatis* nucleic acids, or nucleic acids from other target microbes), or at least one container for packaging the microbial biomolecule detection reagents and the set of instructions. Exemplary solid supports included in the kits of the invention are optionally selected from, e.g., plate, beads, microbeads, fibers, whiskers, combs, hybridization chips, membranes, single crystals, ceramic layers, self-assembling monolayers, and the like.

In embodiments that include primers, the kits typically further include nucleotide incorporating biocatalysts (e.g., polymerases, ligases, etc.), one or more nucleotides (e.g., labeled nucleotides, etc.), and instructions for amplifying one or more subsequences of target microbial nucleic acids with the primers, biocatalysts, and nucleotides. In certain embodiments, primers comprise at least one label (e.g., a fluorescent dye, a radioisotope, etc.). Suitable labels are described further herein. For example, primers are optionally conjugated with biotin or a biotin derivative. In these embodiments, the kit typically further includes an enzyme conjugated with avidin or an avidin derivative, or streptavidin or a streptavidin derivative, e.g., for effecting the detection of binding between the microbial biomolecule detection reagents (e.g., oligonucleotide probes) of the invention and target nucleic acids or amplicons thereof. In some of these embodiments, the kits further include at least one pyrophosphatase (e.g., a thermostable pyrophosphatase), e.g., for use in minimizing pyrophosphorolysis, uracil N-glycosylase (UNG) (e.g., a thermostable UNG), e.g., for use in applications where protection against carry-over contamination is desirable.

### A PREFERRED KITS

A preferred kit according to the present invention comprises:
- at least one stabilization component comprising at least one chaotropic reagent; and,
- instructions for contacting at least one non-blood-based sample comprising microbial biomolecules with an effective amount of the stabilization component to produce a stabilized non-blood-based sample in which the microbial biomolecules are stable at a temperature of about 20°C or more.

Preferably, the stabilization component of this kit is substantially free of a chelating agent.

The stabilization component of this kit preferably either comprises a stabilization solution or comprises one or more solid materials.

The chaotropic reagent of this kit preferably comprises one or more of: guanidine isothiocyanate or a salt thereof, guanidine hydrochloride or a salt thereof, urea, sodium iodide, potassium iodide, potassium chloride, ammonium thiocyanate, sodium thiocyanate, trifluoroethanol, or sodium perchlorate. Preferably the chaotropic reagent is present in the stabilization component at a concentration of about 5.0M or less. Preferably, this kit comprising at least one container that comprises the stabilization component.

Preferably, this kit further comprises at least one transport medium.

Preferably, this kit further comprises instructions for detecting the microbial biomolecules in the stabilized non-blood-based sample.

Preferably the stabilization component of this kit further comprises one or more of: an organic solvent, a reducing agent, a buffer, or a detergent as described below.

The stabilization component of this kit preferably further comprises at least one organic solvent., which more preferably comprises about 50% or less of a total weight of the stabilization component.

The organic solvent of this kit preferably comprises one or more of: polyethylene glycol, acetone, methanol, ethanol, or dimethylsulfoxide. In case this kit comprises polyethylene glycol it comprises a molecular weight of between about 5000 daltons and about 15000 daltons.

The stabilization component of this kit preferably further comprises at least one reducing agent, which more preferably comprises one or more of: dithiothreitol, dithioerithritol, 2-aminoethanethiol, or 2-mercaptoethanol. Even more preferably the reducing agent is present in the stabilization component at a concentration of about 200mM or less.

The stabilization component of this kit preferably further comprises at least one buffer, which preferably comprises one or more of: tris-(hydroxymethyl)aminoethane, tris-(hydroxymethyl)aminomethane sodium phosphate, bis(2-hydroxyethyl)-imino-tris(hydroxymethyl)methane, sodium acetate, sodium citrate, sodium tetraborate, glycine-sodium hydroxide, N-2-hydroxyethylpiperazine-N'-2-ethanesulfonic acid, 3-[(1,1-dimethyl-hydroxyethyl)amino]-2-hydroxypropanesulfonic acid, 3-(cyclohexylamino)-2-hydroxy-1-propanesulfonic acid, 3-[N,N-bis(hydroxyethyl)amino]-2-hydroxypropanesulfonic acid, N-(2-hydroxyethyl)piperazine-N'-2-hydroxy propanesulfonic acid, 3-(N-morpholino)-2-hydroxypropanesulfonicsulfonic acid, piperazine-N,N'-bis(2-hydroxypropanesulfonic acid), 3-[N-tris-(hydroxymethyl) methyl amino]-2-hydroxypropanesulfonic acid, 2-(N-morpholino)-ethanesulfonic acid, 3-(N-morpholino)propanesulfonic acid, piperazine-N,N'-bis(ethanesulfonic acid), acetate, citrate, hydrochloric acid, potassium chloride, glycine, potassium hydrogen phthalate, citric acid, acetic acid, sodium hydroxide, disodium hydrogen phthalate, sodium dihydrogen orthophosphate, dipotassium hydrogen phthalate, potassium dihydrogen orthophosphate, barbitone sodium, sodium tetraborate, sodium carbonate, or sodium hydrogen carbonate. Preferably, the buffer is present in the stabilization component of this kit at a concentration of about 100mM or less.

The stabilization component of this kit preferably further comprises at least one detergent, which more preferably comprises one or more of: polyoxyethylenesorbitan monolaurate, sarcosine, sodium dodecyl sulfate, or α-[4-(1,1,3,3-tetramethylbutyl)phenyl] -omega-hydroxypoly(oxy-1,2-ethanediyl). Preferably, this detergent comprises about 1% or less of a total weight of the stabilization component.

### VI. EXAMPLES

It is understood that the examples and embodiments described herein are for illustrative purposes only and that various modifications or changes in light thereof will be suggested to persons skilled in the art and are to be included within the spirit and purview of this application and scope of the appended claims.

### EXAMPLE 1: STABILITY OF N. GONORRHOEAE IN URINE SAMPLES UNDER VARIED CONDITIONS OVER TIME

This example relates to an analysis of N. *gonorrhoeae* stability in urine samples under different conditions over time. More specifically, N. *gonorrhoeae* activity levels in patient urine specimens were monitored for seven days at 2-8°C and at 30°C. Some of the urine samples were left untreated, that is, no stabilization components were added to the samples. A lysis reagent or stabilization component of the invention was added to other samples such that 10% of the total weight of each sample was the lysis reagent.

The lysis reagent contained the following components:
4.2M Guanidine Isothiocyanate;
3.5% Polyethylene Glycol (MW 10,000);
160mM Dithiothreitol;
50mM Tris (pH 7.5); and
0.1% TWEEN® 20.

Two additional conditions were also examined using DNA/RNA PROTECT™ (Sierra Diagnostics, Inc., Sonora, CA, USA) at final urine concentrations of 10% and 40% by weight. As referred to above, a sample is stabilized when the activity level of the component under stability in the sample deviates by less than about 20% whether the sample is maintained at about 30°C or between about 2-8°C.

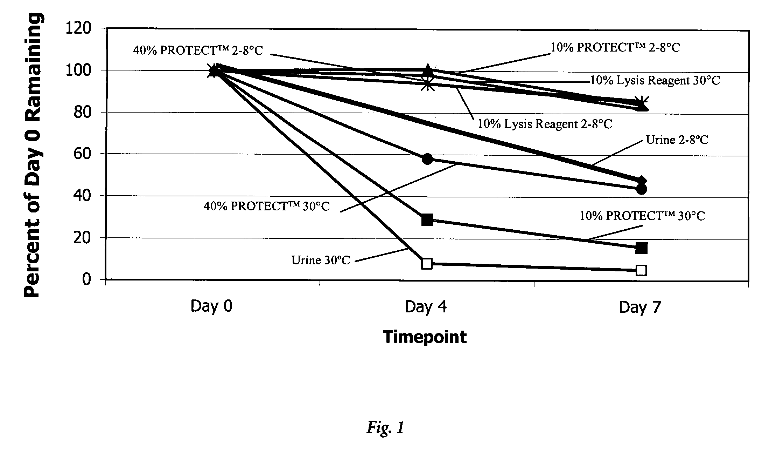

Figure 1 is a graph that shows N. *gonorrhoeae* activity levels detected in the urine samples under the conditions described above. In particular, the abscissa of the graph shown in Figure 1 represents the percentage of the initial N. *gonorrhoeae* activity level remaining, while the ordinate represents the timepoint in days. As shown in Figure 1:
(1) if urine is left untreated, endogenous N. *gonorrhoeae* DNA is rapidly degraded at 30°C, leaving approximately 5% of the zero timepoint activity at day 7 (Urine 30°C);
(2) if urine is left untreated, endogenous N. *gonorrhoeae* DNA is degraded at 2-8°C, leaving approximately 48% of the zero timepoint activity at day 7 (Urine 2-8°C);
(3) if urine is treated with 10% lysis reagent, endogenous N. *gonorrhoeae* DNA at 30°C retains approximately 82% of the zero timepoint activity at day 7 (10% Lysis Reagent 30°C);
(4) if urine is treated with 10% lysis reagent, endogenous N. *gonorrhoeae* DNA at 2-8°C retains approximately 86% of the zero timepoint activity at day 7 (10% Lysis Reagent 2-8°C);
(5) if urine is treated with 10% DNA/RNA PROTECT™, endogenous N. *gonorrhoeae* DNA is rapidly degraded at 30°C, leaving approximately 16% of the zero timepoint activity at day 7 (10% PROTECT™ 30°C);
(6) if urine is treated with 10% DNA/RNA PROTECT™, endogenous N. *gonorrhoeae* DNA at 2-8°C retains approximately 84% of the zero timepoint activity at day 7 (10% PROTECT™ 2-8°C);
(7) if urine is treated with 40% DNA/RNA PROTECT™, endogenous N. *gonorrhoeae* DNA is degraded at 30°C, leaving approximately 44% of the zero timepoint activity at day 7 (40% PROTECT™ 30°C); and
(8) if urine is treated with 40% DNA/RNA PROTECT™, endogenous N. *gonorrhoeae* DNA at 2-8°C retains approximately 85% of the zero timepoint activity at day 7 (40% PROTECT™ 2-8°C).

Accordingly, the results depicted in Figure 1 indicate a loss of stability as defined herein for all conditions with the exception of urine stabilized with 10% lysis reagent, which had activity levels that deviated from one another by less than 20% over the seven days irrespective of whether the samples were maintained at 30°C or at 2-8°C.

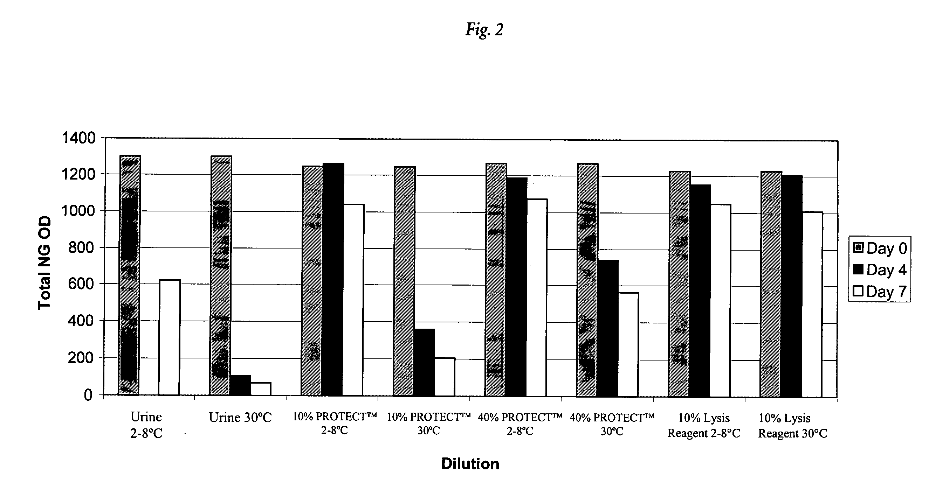

To further illustrate, Figure 2 is a graph of the data presented in Figure 1 that shows actual total optical density [OD] readings. More specifically, the abscissa of the graph of Figure 2 represents total N. *gonorrhoeae* (NG) OD in the samples, while the ordinate represents sample dilution.

### EXAMPLE 2: DETECTION OF N. GONORRHOEAE/C. TRACHOMATIS IN CLINICAL SAMPLES

This prophetic example describes a protocol for the detection of N. *gonorrhoeae* and C. *trachomatis* in clinical samples.

### Clinical Samples

Endocervical swab specimens from women and urethral swab specimens from men are collected by standard procedures known in the art. Swabs are inoculated into suitable culture transport media (e.g., 2SP, M-4 (Microtest, Inc., Atlanta, GA), Bartel's chlamydial (Intracel Corp., Issaquah, WA), etc.), which is then used for PCR analysis (see also, Van der Pol et al. (2000) J. Clin. Microbiol. 38:1105-1112, which is incorporated by reference). Typically, cell cultures are also inoculated. To stabilize biomolecules in the specimens, the lysis reagent (described in Example 1) is added such that 10% of the total weight of each sample is the lysis reagent. The specimens are typically vortexed with the swab still in the tube, which is then generally stored at room temperature for up to 7 days postcollection and then processed for PCR analysis.

Optionally, 50 ml aliquots of first-catch urine is also collected from both men and women. Female urine specimens are collected either before or after swab collection. Male urine specimens are collected after the urethral swab specimens have been obtained. As above, to stabilize biomolecules in the specimens, the lysis reagent is added such that 10% of the total weight of each urine sample is the lysis reagent. Urine specimens are then typically stored at room temperature for up to 7 days from the time of collection until it is processed for PCR analysis.

### PCR Analysis

Each specimen is typically further processed and subjected to either or both the AMPLICOR® and COBAS AMPLICOR® tests as described in the manufacturer's package inserts. For each processed specimen, the C. *trachomatis, N. gonorrhoeae,* and internal control (IC) target DNAs are simultaneously amplified in a single stabilized non-blood-based sample that contains at least two primer pairs, at least one pair specific for C. *trachomatis* and at least one pair specific for N. *gonorrhoeae.* The resulting amplification products are generally captured separately and detected colorimetrically by hybridization to microwell plates (AMPLICOR® format) (Crotchfelt et al. (1997) "Detection of *Neisseria gonorrhoeae* and *Chlamydia trachomatis* in genitourinary specimens from men and women by a coamplification PCR assay," J. Clin. Microbiol. 35:1536-1540, which is incorporated by reference) or to magnetic microparticles (COBAS AMPLICOR® format) coated with N. *gonorrhoeae-, C. trachomatis-,* and IC-specific oligonucleotide probes. The COBAS AMPLICOR® analyzer automatically performs all of the amplification, hybridization, and detection steps (DiDomenico et al. (1996) "COBAS AMPLICOR™: a fully automated RNA and DNA amplification and detection system for routine diagnostic PCR," Clin. Chem. 42:1915-1923, Jungkind et al. (1996) "Evaluation of automated COBAS AMPLICOR PCR system for detection of several infectious agents and its impact on laboratory management," J. Clin. Microbiol. 34:2778-2783, which are both incorporated by reference). In the AMPLICOR® format, amplification is performed, e.g., with a GeneAmp® PCR System 9700 thermal cycler (Perkin-Elmer, Norwalk, CT) and hybridization and detection are performed manually (Loeffelholz et al. (1992) "Detection of *Chlamydia trachomatis* in endocervical specimens by polymerase chain reaction," J. Clin. Microbiol. 30:2847-2851, which is incorporated by reference).

### Data Analysis

Specimens yielding signals above a positive cutoff (optical density [OD] of 2.0 (A₆₆₀) for *C. trachomatis* and OD of 3.5 (A₆₆₀) for N. *gonorrhoeae)* are typically interpreted as positive for the particular organism, regardless of the IC result. Specimens yielding N. *gonorrhoeae* or C. *trachomatis* signals below a negative cutoff (e.g., OD of 0.2) are typically interpreted as negative for the particular organism, provided that the IC signal is above the assigned cutoff (e.g., OD of 0.2) and the test considered valid. Specimens *yielding N.gonorrhoeae* or C. *trachomatis* signals below the cutoff values for both N. *gonorrhoeae* or *C*. *trachomatis* and IC are generally interpreted as inhibitory. Inhibitory specimens are typically retested by processing an aliquot of the original specimen. The repeat test results are classified using the above criteria.

Specimens yielding results between the negative and positive cutoffs (≥ 0.2, < 3.5 for *N*. *gonorrhoeae* and ≥ 0.2, < 2.0 for C. *trachomatis*) are typically considered equivocal for the particular organism, regardless of the IC signal. Equivocal results are generally resolved by processing an aliquot of the original specimen, retesting in duplicate and comparing the results to the initial test. These specimens are typically interpreted as positive for the particular organism if at least two valid tests yields an N. *gonorrhoeae* or *C. trachomatis* OD of ≥ 2.0 for the particular organism. These specimens are generally interpreted as negative for the particular organism if the two repeat tests yield N. *gonorrhoeae* or *C*.*trachomatis* signals of <0.2 OD for the particular organism, provided that the IC signals are above the assigned cutoff. If the two repeat tests yield an N. *gonorrhoeae* or C. *trachomatis* OD of <0.2 for the particular organism and the IC signal is below the assigned cutoff for either of the duplicate repeat tests, the specimen is generally interpreted as inhibitory.

While the foregoing invention has been described in some detail for purposes of clarity and understanding, it will be clear to one skilled in the art from a reading of this disclosure that various changes in form and detail can be made without departing from the true scope of the invention. For example, all the techniques and apparatus described above can be used in various combinations. All publications, patents, patent applications, and/or other documents cited in this application are incorporated by reference in their entirety for all purposes to the same extent as if each individual publication, patent, patent application, and/or other document were individually indicated to be incorporated by reference for all purposes.

## Claims

1. A stabilized non-blood-based sample, comprising:
- at least one non-blood-based sample that comprises microbial biomolecules; and,
- an effective amount of at least one stabilization component comprising at least one chaotropic reagent,
wherein the microbial biomolecules in the stabilized non-blood-based sample are stable at a temperature of about 20°C or more.

2. The stabilized non-blood-based sample of claim 1, wherein the effective amount of the stabilization component comprises about 45% or less of a total weight of the stabilized non-blood-based sample.

3. The stabilized non-blood-based sample of claim 1, wherein the non-blood-based sample comprises urine and the effective amount of the stabilization component comprises about 15% or less of a total weight of the stabilized non-blood-based sample.

4. The stabilized non-blood-based sample of claim 1, wherein the stabilization component is substantially free of a chelating agent.

5. The stabilized non-blood-based sample of claim 1, wherein the chaotropic reagent comprises one or more of: guanidine isothiocyanate or a salt thereof, guanidine hydrochloride or a salt thereof, urea, sodium iodide, potassium iodide, potassium chloride, ammonium thiocyanate, sodium thiocyanate, trifluoroethanol, or sodium perchlorate.

6. The stabilized non-blood-based sample of claim 1, wherein the chaotropic reagent is present in the stabilized non-blood-based sample at a concentration of about 5.0M or less.

7. The stabilized non-blood-based sample of claim 1, wherein the non-blood-based sample comprises urine and the chaotropic reagent is present in the stabilized non-blood-based sample at a concentration of about 0.5M or less.

8. The stabilized non-blood-based sample of claim 1, wherein levels of the microbial biomolecules in the stabilized non-blood-based sample deviate from one another by less than about 15% over at least about a seven day period whether a temperature of the stabilized non-blood-based sample is maintained at about 30°C or between about 8°C and about 2°C.

9. A reaction mixture, comprising:
- a stabilized non-blood based sample comprising:
- at least one non-blood-based sample comprising one or more microbial nucleic acids; and
- an effective amount of at least one stabilization component comprising at least one chaotropic reagent, wherein the stabilization component stabilizes the microbial nucleic acids at a temperature of about 20°C or more; and,
- at least one nucleic acid amplification reagent selected from the group consisting of: a primer nucleic acid, a probe nucleic acid, a nucleotide incorporating biocatalyst, and extendible nucleotides.

10. The reaction mixture of claim 9, wherein the effective amount of the stabilization component comprises about 45% or less of a total weight of the stabilized non-blood-based sample.

11. The reaction mixture of claim 9, wherein the stabilization component further comprises one or more of: an organic solvent, a reducing agent, a buffer, or a detergent.

12. The reaction mixture of claim 9, wherein the stabilization component is substantially free of a chelating agent.

13. A method of stabilizing microbial biomolecules in non-blood-based samples, the method comprising:
- providing at least one non-blood-based sample comprising the microbial biomolecules; and,
- contacting the non-blood-based sample with an effective amount of at least one stabilization component comprising at least one chaotropic reagent to produce a stabilized non-blood-based sample, wherein the stabilization component stabilizes the microbial biomolecules at a temperature of about 20°C or more.

14. The method of claim 13, wherein the stabilization component is substantially free of a chelating agent.

15. The method of claim 13, wherein the effective amount of the stabilization component comprises about 45% or less of a total weight of the stabilized non-blood-based sample.

16. The method of claim 13, wherein the non-blood-based sample comprises urine and the effective amount of the stabilization component comprises about 15% or less of a total weight of the stabilized non-blood-based sample.

17. The method of claim 13, wherein the chaotropic reagent comprises one or more of: guanidine isothiocyanate or a salt thereof, guanidine hydrochloride or a salt thereof, urea, sodium iodide, potassium iodide, potassium chloride, ammonium thiocyanate, sodium thiocyanate, trifluoroethanol, or sodium perchlorate.

18. The method of claim 13, wherein levels of the microbial biomolecules in the stabilized non-blood-based sample deviate from one another by less than about 15% over at least about a seven day period whether a temperature of the stabilized non-blood-based sample is maintained at about 30°C or between about 8°C and about 2°C.

19. The method of claim 13, further comprising:
- isolating one or more of the microbial biomolecules after the contacting step.

20. The method of claim 13 or 19, further comprising:
- detecting one or more of the microbial biomolecules.

21. The method of claim 20, wherein the detecting step comprises:
- amplifying a detectable signal produced by the microbial biomolecules, or by a microbial biomolecule detection reagent that detectably binds to at least one of the microbial biomolecules, to produce an amplified signal; and,
- detecting the amplified signal.

22. The method of claim 20, wherein the microbial biomolecules comprise nucleic acids and the detecting step comprises:
- amplifying at least a segment of one or more of the nucleic acids to produce amplified nucleic acids; and,
- detecting one or more of the amplified nucleic acids during or after the amplifying step.

23. The stabilized non-blood-based sample produced by the method of any of claims 13 to 22.

24. A kit, comprising:
- at least one stabilization component comprising at least one chaotropic reagent; and,
- instructions for contacting at least one non-blood-based sample comprising microbial biomolecules with an effective amount of the stabilization component to produce a stabilized non-blood-based sample in which the microbial biomolecules are stable at a temperature of about 20°C or more.

25. The kit of claim 24, wherein the stabilization component is substantially free of a chelating agent.

26. The kit of claim 24, wherein the chaotropic reagent comprises one or more of: guanidine isothiocyanate or a salt thereof, guanidine hydrochloride or a salt thereof, urea, sodium iodide, potassium iodide, potassium chloride, ammonium thiocyanate, sodium thiocyanate, trifluoroethanol, or sodium perchlorate.

27. The kit of claim 24, wherein the chaotropic reagent is present in the stabilization component at a concentration of about 5.0M or less.
